(19) Europäisches Patentamt
European Patent Office
Office européen des brevets

(11) EP 2 817 284 B1

(12) **EUROPÄISCHE PATENTSCHRIFT**

(45) Veröffentlichungstag und Bekanntmachung des Hinweises auf die Patenterteilung:
**25.10.2017 Patentblatt 2017/43**

(21) Anmeldenummer: **13703588.7**

(22) Anmeldetag: **11.02.2013**

(51) Int Cl.:
***C07C 45/50*** (2006.01) ***C07C 45/78*** (2006.01)
***C07C 45/82*** (2006.01) ***C07C 47/02*** (2006.01)
***B01D 61/02*** (2006.01)

(86) Internationale Anmeldenummer:
**PCT/EP2013/052633**

(87) Internationale Veröffentlichungsnummer:
**WO 2013/124176 (29.08.2013 Gazette 2013/35)**

(54) **VERFAHREN UND VORRICHTUNG ZUR HYDROFORMYLIERUNG VON ISOBUTEN UND ZUM AUFTRENNEN DES PRODUKTGEMISCHES**

METHOD AND DEVICE FOR THE HYDROFORMYLATION OF ISOBUTENE AND FOR THE SEPARATION OF THE PRODUCT MIXTURE

PROCÉDÉ ET DISPOSITIF DESTINÉS À L'HYDROFORMYLATION D'ISOBUTÈNE ET À LA SÉPARATION DU MÉLANGE DE PRODUITS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.02.2012 DE 102012202779**

(43) Veröffentlichungstag der Anmeldung:
**31.12.2014 Patentblatt 2015/01**

(73) Patentinhaber: **Evonik Degussa GmbH**
**45128 Essen (DE)**

(72) Erfinder:
- **BAUMGARTEN, Goetz**
  **45721 Haltern am See (DE)**
- **GRASS, Michael**
  **45721 Haltern am See (DE)**
- **KAIZIK, Alfred**
  **45772 Marl (DE)**
- **WINTERBERG, Markus**
  **45711 Datteln (DE)**
- **LUEKEN, Hans-Gerd**
  **45770 Marl (DE)**
- **HAMERS, Bart**
  **5961 VG Horst (NL)**
- **PRISKE, Markus**
  **Mobile, AL 36609 (US)**
- **FRIDAG, Dirk**
  **45721 Haltern am See (DE)**
- **FRANKE, Robert**
  **45772 Marl (DE)**
- **HESS, Dieter**
  **45770 Marl (DE)**

(56) Entgegenhaltungen:
**WO-A1-2008/006633 DE-A1-102005 046 250**

- **Siavash Darvishmanesh ET AL: "Physicochemical Characterization of Transport in Nanosized Membrane Structures", CHEMPHYSCHEM - A EUROPEAN JOURNAL OF CHEMICAL PHYSICS & PHYSICALCHEMISTRY., vol. 11, no. 2, 1 February 2010 (2010-02-01), pages 404-411, XP055267256, DE ISSN: 1439-4235, DOI: 10.1002/cphc.200900641**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 817 284 B1

## Beschreibung

Die vorliegende Erfindung betrifft ein Verfahren zum Herstellen eines Produktgemisches durch technische Hydroformylierung eines Isobuten enthaltenden Kohlenwasserstoffstroms und zum Auftrennen des erhaltenen Produktgemisches. Unter Hydroformylierung wird im technischen Bereich im Allgemeinen die Umsetzung eines Olefins mit Synthesegas (hauptsächlich aus Kohlenmonoxid und Wasserstoff bestehendes Gasgemisch), gewöhnlich unter Druck und in Gegenwart eines Übergangsmetallkomplex-Katalysators, zu einem gegenüber dem Olefin um ein Kohlenstoff-Atom verlängerten Aldehyd verstanden.

Grundlegende Einführungen in die Hydroformylierung bieten: Falbe, Jürgen: New Syntheses with Carbon Monoxide. Springer Verlag 1980, Berlin, Heidelberg, New York und Pruett, Roy L.: Hydroformylation. Advances in Organometallic Chemistry Vol. 17, Pages 1-60, 1979.

Allgemein dient die Hydroformylierung zur Herstellung von höheren Aldehyden. Höhere Aldehyde, insbesondere solche mit 3 bis 25 Kohlenstoffatome, werden zum Beispiel als Synthesevorstufen, zur Herstellung von Carbonsäuren und als Riechstoffe genutzt. Technisch werden sie oft durch katalytische Hydrierung in die entsprechenden Alkohole überführt, die wiederum für die Herstellung von Weichmachern und Detergentien dienen. Aufgrund der großtechnischen Bedeutung der Hydroformylierungsprodukte wird die Oxo-Reaktion im industriellen Maßstab durchgeführt.

Durch Hydroformylierung des $C_4$-Olefins Isobuten entsteht Isovaleraldehyd, welches im Folgenden als 3-Methylbutanal oder kurz 3MBA bezeichnet wird. 3MBA wird zur Herstellung von Riech- und Aromastoffen sowie als Synthesevorstufe verwendet.

In der großtechnischen Hydroformylierung werden heute phosphororganische Metall-Komplexkatalysatoren auf Cobalt- oder Rhodiumbasis verwendet. Die Katalysatoren werden homogen in der flüssigen Hydroformylierungsmischung gelöst. Im Rahmen der Ausscheidung des Zielprodukts (der Aldehyde) aus dem Hydroformylierungsgemisch ist auch der Homogenkatalysator aus dem Hydroformylierungsgemisch schonend abzutrennen, da der Komplexkatalysator vergleichsweise empfindlich auf Zustandsänderungen reagiert und seine Aktivität verlieren könnte. Traditionell wird der Katalysator destillativ aus den Hydroformylierungsgemisch abgeschieden. Um die Gefahr der Desaktivierung zu senken und den Energieverbrauch des Prozesses zu senken gibt es in jüngerer Zeit Bestrebungen, den homogen gelösten Katalysator mit Hilfe von Membrantechnologie (Nanofiltration) aus dem Hydroformylierungsgemisch abzutrennen.

Die Grundlagen der Membrangestützten, organophilen Nanofiltration zur Abscheidung von homogen gelösten Katalysatorkomplexen aus Hydroformylierungsmischungen beschreiben Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Auch die EP1931472B1 beschäftigt sich allgemein mit der organophilen Nanofiltration zur Abscheidung homogen gelösten Katalysatorkomplexen aus Hydroformylierungsmischungen.

Ein Verfahren zur Herstellung von 3-Methylbutanal durch Hydroformylierung von Isobuten wird in der Patentanmeldung WO2008006633 beschrieben.

Die katalysierte Hydroformylierung von Olefinen zu den entsprechenden Aldehyden erfolgt üblicherweise in homogener, flüssiger Phase, d.h. Katalysator, Olefin und Produkte liegen in einer Phase vor, wobei der Übergangsmetallkomplex-Katalysator homogen im flüssigen Reaktionsgemisch gelöst ist, welches auch das zu hydroformylierende Olefin und Produkte der Hydroformylierung umfasst. Als Produkte der Hydroformylierung werden neben dem besagten Aldehyd als primärem Produkt 3-Methylbutanal typischerweise auch höher siedende Folgeprodukte (üblicherweise als Hochsieder bezeichnet) gebildet. Zusätzlich kann im Reaktionsgemisch ein inertes Lösungsmittel für den Übergangsmetallkomplex-Katalysator, wie beispielsweise Dioctylphthalat oder Diisononylphthalat oder Isononyl benzoate oder Mischungen daraus, vorhanden sein.

Unter "Hochsiedern" werden hier Stoffe verstanden, die bei einer höheren Temperatur sieden und höhere Molmassen aufweisen als das primäre Hydroformylierungsprodukt (Aldehyd mit einem Kohlenstoff-Atom mehr als das eingesetzte Olefin) und der daraus durch Hydrierung erhaltene Alkohol. Hochsieder entstehen durch Folgereaktionen aus dem primären Hydroformylierungsprodukt. Zu den typischerweise bei technischen Hydroformylierungen entstehenden Hochsiedern gehören Aldolisierungsprodukte und Acetalisierungsprodukte sowie Ester, die durch Reaktion von Alkoholen und Säuren entstehen, wobei die Alkohole und Säuren insbesondere durch Disproportionierung von Aldehyden gebildet werden.

Bei der technischen Hydroformylierung von Isobuten entsteht typischerweise ein Produktgemisch, das neben dem primären Produkt 3-Methylbutanal, welches das Zielprodukt der technischen Hydroformylierung von Isobuten ist, Folgeprodukte in Form von Hochsiedern und den Übergangsmetallkomplex-Katalysator und dessen freie Liganden umfasst. Je nach Umsatzleistung der Reaktion kann das aus dem Reaktor entnommene Produktgemisch auch nicht umgesetztes Edukt enthalten, also Isobuten, Wasserstoff oder Kohlenmonoxid. Um die Reinheit des primären Produkts 3-Methylbutanal zu erhöhen und den Übergangsmetallkomplex-Katalysator zurückzugewinnen, ist es erforderlich, die

Bestandteile 3MBA, Folgeprodukte und Katalysator und der gegebenenfalls nicht umgesetzten Einsatzstoffe des bei der Hydroformylierung erhaltenen Produktgemisches voneinander zu trennen.

Ein Verfahren zum Anreichern eines Homogenkatalysators aus einem Prozessstrom ist aus der WO2010097376A1 bekannt. Der Prozessstrom stammt dabei beispielsweise aus einem Verfahren zur Hydroformylierung von Olefinen, bevorzugt mit 2 bis 25 Kohlenstoffatomen, zu den entsprechenden Aldehyden, insbesondere Isononanal und Isotridecanal. Die Anreicherung des Homogenkatalysators aus dem Prozessstrom erfolgt in einer solchen Weise, dass der Prozessstrom über mindestens eine Nanofiltrationsmembran geführt wird, die ganz oder teilweise aus einem Polymer besteht, das planare Polymereinheiten aufweist, die über einen starren Linker miteinander verbunden sind, wobei der Linker in sich verdreht ist, so dass mindestens ein planare Polymereinheit über den Linker in einer nicht coplanaren Anordnung mit mindestens einer zweiten planaren Polymereinheit verbunden ist. Bei der Membranfiltration verbleibt das Katalysatorsystem im Retentat, während die Hochsieder mit dem Permeat abgetrennt werden. Vor dem Trennen durch Membranfiltration erfolgt bevorzugt eine destillative Auftrennung des Austrags des Hydroformylierungsreaktors in ein Destillat, das nicht umgesetzte Olefine und die gewünschten Aldehyde enthält, und ein Sumpfprodukt, das Hochsieder und das Katalysatorsystem enthält.

Ein anderes Verfahren zur Abtrennung und teilweisen Rückführung eines Übergangsmetallkomplex-Katalysators aus einem Reaktionsgemisch, z.B. aus dem bei einer technischen Hydroformylierung erhaltenen Reaktionsgemisch, ist aus WO2010097428 bekannt. Dieses Verfahren basiert auf einer Kombination einer mindestens einstufigen Membrantrennung und einer Adsorption. Dabei wird ein katalysatorhaltiger Strom über mindestens einen einstufigen Membrantrennschritt in einen an Übergangsmetallkomplex-Katalysator angereicherten Retentatstrom und einen an Übergangsmetallkomplex-Katalysator abgereicherten Permeatstrom aufgetrennt. Der an Übergangsmetallkomplex-Katalysator angereicherte Retentatstrom wird in den Reaktor zurückgeführt. Der an Übergangsmetallkomplex-Katalysator abgereicherte Permeatstrom wird einem Adsorptionsschritt zugeführt, in welchem eine weitere Abtrennung des Übergangsmetallkomplex-Katalysators aus dem Permeatstrom erfolgt.

Diese und andere aus dem Stand der Technik bekannte Verfahren zielen darauf ab, den Übergangsmetallkomplex-Katalysator so weit wie möglich von den Hochsiedern abzutrennen, um eine möglichst vollständige Rückgewinnung des Übergangsmetallkomplex-Katalysators zu erreichen. Der abgetrennte Katalysator kann - gegebenenfalls nach erforderlicher Aufarbeitung - in den Hydroformylierungsreaktor zurückgeführt werden, so dass die Wirtschaftlichkeit des Prozesses verbessert werden kann.

Die Bildung von Hochsiedern vermindert die Ausbeute der Hydroformylierung in Bezug auf das primäre Produkt 3-Methylbutanal und beeinträchtigt daher die Wirtschaftlichkeit des Verfahrens. Im Interesse einer verbesserten Ausnutzung der eingesetzten Rohstoffe (Isobuten; Synthesegas) und des eingesetzten Übergangsmetallkomplex-Katalysators ist es daher wünschenswert, den Anteil an Hochsiedern im Produktgemisch möglichst gering zu halten.

Die Aufgabe der vorliegenden Erfindung besteht somit darin, ein Verfahren und eine Vorrichtung zum Herstellen eines Produktgemisches durch technische Hydroformylierung eines Isobuten enthaltenden Kohlenwasserstoffstroms und zum Auftrennen des erhaltenen Produktgemisches anzugeben, die es ermöglichen, den Anteil an Hochsiedern im Produktgemisch möglichst gering zu halten und damit die Ausbeute der Reaktion zu steigern.

Diese Aufgabe wird gelöst durch ein erfindungsgemäßes Verfahren zum Herstellen eines Produktgemisches durch technische Hydroformylierung eines Isobuten enthaltenden Kohlenwasserstoffstroms und zum Auftrennen des erhaltenen Produktgemisches, mit folgenden Schritten:

a) Hydroformylieren des Isobuten enthaltenden Kohlenwasserstoffstroms in einem Hydroformylierungsreaktor in Gegenwart eines Übergangsmetallkomplex-Katalysators, so dass ein Produktgemisch erhalten wird, das zumindest 3-Methylbutanal, Folgeprodukte in Form von Hochsiedern und 3-Methylbutansäure, sowie den Übergangsmetallkomplex-Katalysator nebst dessen freie Liganden umfasst,

b) Trennen des Produktgemisches mittels einer eine oder mehrere Membrantrennstufen umfassenden Nanofiltrationseinrichtung, sodass im resultierenden Retentat der Nanofiltrationseinrichtung der Übergangsmetallkomplex-Katalysator und dessen freie Liganden gegenüber 3-Methylbutanal und 3-Methylbutansäure angereichert sind, und sodass im resultierenden Permeat der Nanofiltrationseinrichtung 3-Methylbutanal und 3-Methylbutansäure jeweils gegenüber dem Übergangsmetallkomplex-Katalysator angereichert sind, wobei im resultierendem Retentat die Konzentration der 3-Methylbutansäure geringer ist als im Permeat,

c) Trennen des resultierenden Permeats der Nanofiltrationseinrichtung mittels einer eine oder mehrere Trennstufen umfassenden thermischen Trenneinrichtung in zumindest eine erste Fraktion und eine zweite Fraktion, wobei die erste Fraktion eine höhere Konzentration an 3-Methylbutanal als die zweite Fraktion und eine geringere Konzentration an Folgeprodukten in Form von Hochsiedern und 3-Methylbutansäure als die zweite Fraktion enthält,

d) Rückführen zumindest eines Teilstroms des resultierenden Retentats der Nanofiltrationseinrichtung in den Hydroformylierungsreaktor, ferner umfassend den Schritt

- Überwachen der Konzentration der 3-Methylbutansäure im resultierenden Retentat (3) der Nanofiltrationseinrichtung, und vorzugsweise auch im Produktgemisch (2) und/oder im resultierenden Permeat (4) der Nanofiltrationseinrichtung.

Zur Lösung der gestellten Aufgabe war es zunächst erforderlich, die einzelnen an der Bildung der Hochsieder beteiligten Spezies zu identifizieren und die Kinetik der involvierten Reaktionen aufzuklären. In eigenen Untersuchungen zur Aufklärung der Kinetik der Bildung von Hochsiedem bei der Hydroformylierung von Isobuten zu 3-Methylbutanal wurde überraschenderweise festgestellt, dass als Oxidationsprodukt des 3-Methylbutanals gebildete 3-Methylbutansäure eine kritische Rolle bei der Bildung von Hochsiedern spielt. Die Bildung von Hochsiedern beginnt mit der Aldolkondensation von 3-Methylbutanal:

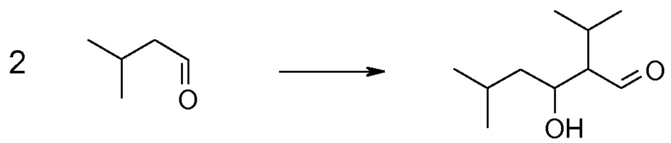

Das Aldolkondensat kann durch Reaktion mit dem primären Produkt 3-Methylbutanal zu einem zweiwertigen $C_{10}$-Alkohol reduziert werden, wobei das primäre Produkt 3-Methylbutanal zur 3-Methylbutansäure oxidiert wird:

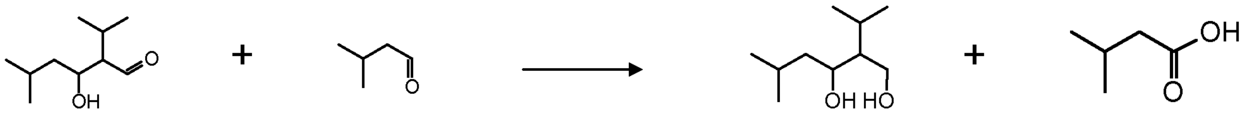

Durch Veresterung des $C_{10}$-Alkohols mit 3-Methylbutansäure entsteht dann in einer Tischtschenko-Reaktion $C_{15}$-Hochsieder:

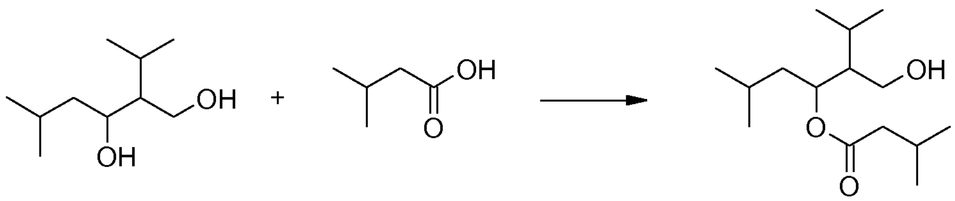

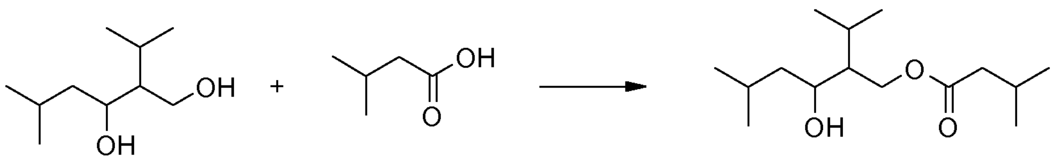

Durch weitere Veresterung des $C_{15}$-Hochsieders mit 3-Methylbutansäure kann schließlich auch ein $C_{20}$-Hochiseder entstehen:

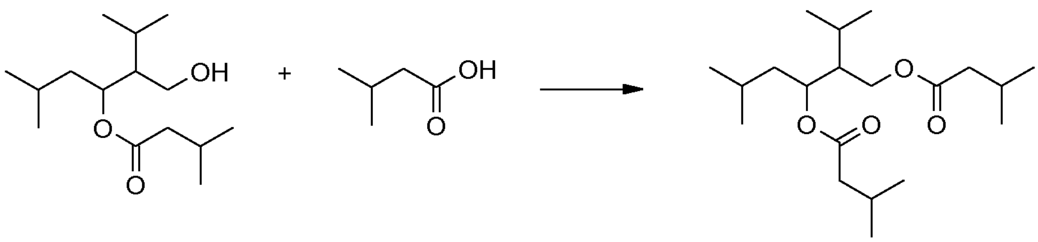

Die Bildung der $C_{15}$- und $C_{20}$-Hochsieder erfolgt also in entscheidendem Maße weitgehend über Reaktionen unter Beteiligung von 3-Methylbutansäure. 3-Methylbutansäure wird im Folgenden gelegentlich mit 3MBS abgekürzt.

Diese Erkenntnis ist die Grundlage der vorliegenden Erfindung: Die erfindungsgemäße Lösung der oben erwähnten Aufgabe, den Anteil an Hochsiedern im resultierenden Produktgemisch der Hydroformylierung möglichst gering zu halten, besteht primär darin, 3-Methylbutansäure möglichst weitgehend von dem in den Hydroformylierungsreaktor

zurückzuführenden Übergangsmetallkomplex-Katalysator abzutrennen. Dies lässt sich erreichen, indem bei dem Schritt des Trennens des resultierenden Produktgemisches der Hydroformylierung der in den Hydroformylierungsreaktor zurückzuführende Übergangsmetallkomplex-Katalysator im resultierenden Retentat der Nanofiltrationseinrichtung angereichert wird, während die Folgeprodukte der Hydroformylierungsreaktion, insbesondere 3MBS, im resultierenden Permeat der Nanofiltrationseinrichtung angereichert werden. Überraschenderweise wurde festgestellt, dass sich mittels Nanofiltration, d. h. Trennung des resultierenden Produktgemisches der Hydroformylierung unter Einsatz einer oder mehrerer Nanofiltrationsmembranen, eine weitgehende Abtrennung der der 3-Methylbutansäure vom Übergangsmetallkomplex-Katalysator erreichen lässt. Dazu wird für den Nanofiltrations-Trennschritt des erfindungsgemäßen Verfahrens eine Nanofiltrationseinrichtung eingesetzt, die eine oder mehrere Nanofiltrationsmembranen enthält, die sich durch einen besonders niedrigen Rückhalt für 3-Methylbutansäure auszeichnen. Bevorzugt ist der Rückhalt der Nanofiltrationsmembran für 3-Methylbutansäure -1 oder kleiner, besonders bevorzugt -5 oder kleiner, und insbesondere bevorzugt -10 oder kleiner. Die Definition des Rückhalts einer Membran findet sich weiter unten.

Durch ihre Abtrennung vom in den Hydroformylierungsreaktor zurückzuführenden Übergangsmetallkomplex-Katalysator steht die mit dem Permeat der Nanofiltrationseinrichtung ausgeschleuste 3-Methylbutansäure im Reaktor nicht mehr als Reaktionspartner für die Hochsiederbildung zur Verfügung, so dass wichtige an der Hochsiederbildung beteiligte Reaktionen nicht mehr oder nur noch in geringerem Maße ablaufen. Dadurch wird bezogen auf das primäre Produkt 3-Methylbutanal der Verlust reduziert.

Ein wichtiger Aspekt der erfindungsgemäßen Lehre besteht also in der Verwendung einer Nanofiltrationseinrichtung zum Abtrennen des Katalysatorkomplexes aus dem Produktgemisch, welche eine besonders hohe Durchlässigkeit für 3-Methylbutansäure aufweist.

Unter Nanofiltrationseinrichtung ist im Sinne dieser Erfindung ein Trennapparat zu verstehen, der seine Trennaufgabe ausschließlich mit Hilfe von Membranen bewerkstelligt, wobei mindestens eine der Membranen eine Nanofiltrationsmembran darstellt. Die Nanofiltrationseinrichtung kann eine oder mehrere Membrantrennstufen umfassen, dementsprechend kann die Nanofiltrationseinrichtung ein oder mehrstufig arbeiten. Jede Membrantrennstufe weist dabei drei Anschlüsse auf, einen Zulauf (Feed) und zwei Abläufe, nämlich Retentat und Permeat. Im Permeat reichern sich die Bestandteile des Zulaufes an, welche die Membran überwinden, währenddessen sich im Retentat die Stoffe anreichern, welche von der Membran zurückgehalten werden. Unter dem resultierenden Permeat bzw. dem resultierenden Retentat sind die beiden Abläufe der Nanofiltrationseinrichtung an ihren Schnittstellen zu den übrigen Komponenten der Vorrichtung zur Durchführung des erfindungsgemäßen Verfahrens zu verstehen. Sofern die Nanofiltrationseinrichtung lediglich einstufig arbeitet, und mithin nur eine Membrantrennstufe aufweist, entspricht das resultierende Permeat bzw. das resultierende Retentat der Nanofiltrationseinrichtung dem Permeat bzw. dem Retentat des einzigen Membrantrennstufe.

Das Grundwissen des Fachmanns auf dem Gebiet der Membranfiltration beschreiben Melin / Rautenbach: Membranverfahren. Grundlagen der Modul- und Anlagenauslegung. Springer, Berlin Heidelberg 2004.

Nanofiltration ist ein druckgetriebenes Membrantrennverfahren. Die Trenngrenze (engl.: molecular weight cut-off, MWCO; vgl. Y.H. See Toh, X.X. Loh, A. Bismarck, A.G. Livingston, In search of a standard method for the characterisation of organic solvent nanofiltration membranes, J. Membr. Sci, 291(2007)120-125]) liegt im Bereich von 150g/mol bis 2000g/mol. Über diesen Wert lässt sich die Nanofiltarion von anderen Membrantrennverfahren wie Mikrofiltration und Ultrafiltration abgrenzen. Die Trenngrenze ist definiert als die Molmasse eines bevorzugt inerten Indikatorsystems (z. B. Polystyrolstandards oder Alkanstandards bei Toh, Loh, Bismarck und Livingston), bei der eine Membran einen Rückhalt von 90% aufweist. Die genaue Trenngrenze einer Nanofiltrationsmembran ist von der eingesetzten Membran und dem jeweiligen Lösemittel sowie durch die Prozessbedingungen wie Druck und Temperatur bestimmt. Bei der Nanofiltration werden dichte oder poröse Membranen eingesetzt. Nanofiltrationsmembranen zeichnen sich durch einen geringen Rückhalt für niedermolekulare organische Stoffe aus.

Der Rückhalt R einer Membran ist über die örtlichen Konzentrationen einer Komponente i des nicht permeierenden Stroms (Retentat) sowie des durch die Membran permeierenden Stroms (Permeat) bestimmt. Sind Retentat und Permeat entlang der Membran ideal durchmischt, so entsprechen die örtlichen Retentat- und Permeatkonzentrationen den jeweiligen Konzentration des im Summe anfallenden Retentats bzw. Permeats. Der Rückhalt R einer Membran für eine im zugeführten Stoffstrom enthaltene Komponente i ist wie folgt definiert:

$$R = 1 - c_{Pi}/c_{Ri}$$

Dabei ist $C_{Pi}$ die Konzentration der Komponente i im Permeat P und $C_{Ri}$ die Konzentration der Komponente i im Retentat R. Im Grenzfall eines vollständigen Rückhalts der Komponente i durch die Membran ist $C_{Pi}$= 0 und R = 1. Im Fall einer bevorzugten Permeation der Komponente i ist $C_{Pi} > C_{Ri}$, und R < 0.

Bevorzugt umfasst die bei dem erfindungsgemäßen Verfahren einzusetzende Nanofiltrationseinrichtung eine

oder mehrere Nanofiltrationsmembranen, wobei die oder mindestens eine der Nanofiltrationsmembranen einem Rückhalt für 3-Methylbutansäure von -1 oder kleiner, besonders bevorzugt -5 oder kleiner, und insbesondere -10 oder kleiner aufweist. So lässt sich eine weitgehende Ausschleusung der 3-Methylbutansäure aus dem resultierenden Produktgemisch der Hydroformylierung erreichen, das der Nanofiltrationseinrichtung als Zulauf zugeführt wird.

Für die Trennaufgabe geeignet erscheinen Membranen, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan, Silicone, Polyphosphazene, Polyphenylsulfide, Polybenzimidazole, 6.6 Nylon, Polysulfone, Polyaniline, Polyurethane, Acrylonitril/Glycidylmethacrylat (PANGMA), Polytrimethylsilylpropyne, Polymethylpentyne, Polyvinyltrimethylsilan, alpha-Aluminiumoxide, Titaniumoxide, gamma-Aluminiumoxide, Polyphenylenoxid, Siliciumoxide, Zirconiumoxide, mit Silanen hydrophobisierte keramische Membranen, wie sie in DE10308111 beschrieben sind, Polymere mit intrinsischer Mikroporosität (PIM) wie PIM-1 und anderen, wie sie z. B. in EP0781166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranes mit Füllstoffen wie z. B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Bevorzugt werden Membranen eingesetzt, die als trennaktive Schicht eine Polymerschicht aus Polydimethylsiloxan, Polyimid, Polyamidimid, Acrylonitril/Glycidylmethacrylat (PANGMA), Polyamid oder Polyetheretherketon aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich.

In eigenen Untersuchungen wurde festgestellt, dass sich Nanofiltrationsmembranen umfassend ein oder mehrere Imidgruppen enthaltende Polymere oder bestehend aus einem oder mehreren Imidgruppen enthaltenden Polymeren besonders für die Ausschleusung der 3-Methylbutansäure aus dem Produktgemisch der Hydroformylierung eignen, das der Nanofiltrationseinrichtung als Zulauf zugeführt wird. Vertreter dieser Membranklasse sind insbesondere Membranen aus Polyimid oder Polyamidimid. Beispielsweise lässt sich das thermoplastische Polyimid einsetzen, was unter dem Markennamen Matrimid® bei der Firma Huntsman Advanced Materials GmbH, Basel (Schweiz) erhältlich ist. Nanofiltrationsmembranen aus Polyimid oder Polyamidimid zeichnen sich durch einen besonders geringen Rückhalt für 3-Methylbutansäure aus. Bevorzugt werden daher in der Nanofiltrationseinrichtung eine oder mehrere Nanofiltrationsmembranen umfassend oder bestehend aus einem oder mehreren Imidgruppen enthaltenden Polymeren eingesetzt, wobei das oder die Imid-gruppen enthaltenden Polymere so ausgewählt sind, das der Rückhalt der Nanofiltrationsmembran für 3-Methylbutansäure -1 oder kleiner, bevorzugt -5 oder kleiner und besonders bevorzugt -10 oder kleiner ist. Nanofiltrationsmembranen enthaltend Imid-Gruppen sind im Handel erhältlich, z. B. unter den Produktnamen STARMEM® 122 und 240 von W. R. Grace & Co.-Conn. 7500 Grace Drive Columbia, Md 21044 US oder Membranen der Produktfamilien Puramem® und Duramem® erhältlich von Evonik Industries AG, Essen (Deutschland). Puramem® und Duramem® enthalten Polyimide wie P84 und/oder Matrimid 5218.

Besonders bevorzugt umfasst die bei dem erfindungsgemäßen Verfahren einzusetzende Nanofiltrationseinrichtung eine oder mehrere Nanofiltrationsmembranen, wobei die oder mindestens eine der Nanofiltrationsmembranen einer Trenngrenze im Bereich von 150 bis 2000 g/mol, bevorzugt 200 bis 600 g/mol besonders bevorzugt 350 bis 500 aufweist. Da bei dem erfindungsgemäßen Verfahren ein Rückhalt von 3-Methylbutansäure nicht gewünscht ist, wird erfindungsgemäß vorzugsweise eine Nanofiltrationsmembran eingesetzt, deren Trenngrenze höher ist als die Molmasse von 3-Methylbutansäure (102 g/mol). Da auch die Hochsieder nicht im Retentat zurückgehalten werden sollen, liegt die Trenngrenze bevorzugt oberhalb der Molmasse der Hochsieder (200 bis 350 g/mol) jedoch unterhalb des Katalysators (500 bis 1000g/mol).

Ganz besonders bevorzugt werden in einer in dem erfindungsgemäßen Verfahren einzusetzende Nanofiltrationseinrichtung eine oder mehrere Nanofiltrationsmembranen umfassend oder bestehend aus einem oder mehreren Imidgruppen enthaltenden Polymeren eingesetzt, wobei das oder die Imidgruppen enthaltenden Polymere so ausgewählt sind, dass die Trenngrenze der Nanofiltrationsmembran im Bereich von 150 bis 2000 g/mol liegt.

Weitere für den Erfolg des Trennschritts in der Nanofiltrationseinrichtung relevante Parameter sind die Temperatur, der transmembrane Druck, die Reyonlds-Zahl bei der Überströmung der Nanofiltrationsmembran, und der Partialdruck von Kohlenmonoxid und/oder Wasserstoff. Bevorzugt wird der Schritt des Trennens des durch Hydroformylieren des isobutenhaltigen Kohlenwasserstoffstroms erhaltenen Produktgemisches in der Nanofiltrationseinrichtung

- bei einer Temperatur im Bereich von 10 bis 150 °C
  und/oder
- bei einem transmembranen Druck im Bereich von 0,5 bis 6 MPa

und/oder

- bei einer Reynoldszahl zwischen 55 und 13500, bevorzugt zwischen 100 und 3500 und ganz besonders bevorzugt zwischen 170 und 900,
  und/oder
- in Gegenwart von Kohlenmonoxid und/oder Wasserstoff, bevorzugt bei einem Kohlenmonoxid-Partialdruck von mindestens 200 kPa im Zulauf, im Retentat und im Permeat

durchgeführt.

Vor dem Eintritt in die Nanofiltrationseinrichtung wird das aus dem Hydroformylierungsreaktor austretende resultierende Reaktionsgemisch vorzugsweise zunächst abgekühlt, sofern dies aus Gründen der Membranstabilität oder für die Einstellung der Trenngrenze notwendig ist ,und entspannt, vorzugsweise auf einen Druck von über 200 kPa. Hierbei werden flüchtige Bestandteile wie nicht umgesetztes Isobuten oder Wasserstoff und Kohlenstoffmonoxid teilweise abgetrennt und ggf. zurückgeführt. Mit dem unter dem Entspannungsdruck verbliebenen Synthesegas wird das Produktgemisch umfassend das primäre Produkt 3-Methylbutanal, Folgeprodukte in Form von Hochsiedern und 3-Methylbutansäure und den Übergangsmetallkomplex-Katalysator und dessen freie Liganden und gegebenenfalls unumgesetztes Produkt, einer Hochdruckpumpe zugeführt, die den für den Nanofiltrationsprozess nötigen transmembranen Druck erzeugt. Optional kann der Hochdruckpumpe ein Vorfiltersystem vorgeschaltet sein.

Unter dem transmembranen Druck wird die Druckdifferenz zwischen der Zulaufseite und der Permeatseite der Nanofiltrationsmembran pro Trennstufe verstanden. Diese Druckdifferenz ist die primäre Triebkraft der Membranfiltration.

Um eine Konzentrationsüberhöhung (Konzentrationspolarisation) oder auch die Bildung von Ablagerungen auf der Nanofiltrationsmembran (Membran-Fouling) zu vermeiden, sind beim Trennen in der Nanofiltrationseinrichtung gewisse Strömungsverhältnisse einzuhalten. Es hat sich gezeigt, dass die Konzentrationsüberhöhung sowie die Bildung von Ablagerungen aus einer eine Nanofiltrationsmembran überströmenden Strömung von deren Turbulenz und damit von ihrer Reynolds-Zahl abhängig ist. So ist unabhängig von der Bauart des Membranmoduls darauf zu achten, dass die Reynoldszahl zwischen 55 und 13500, bevorzugt zwischen 100 und 3500 und ganz besonders bevorzugt zwischen 170 und 900 liegt. Dies gilt insbesondere für Stoffsysteme mit einer kinematischen Viskosität kleiner als 10 mPas. Bei diesen Strömungsverhältnissen werden Konzentrationsüberhöhung und Ablagerungen auf ein sinnvolles Maß reduziert.

Die dimensionslose Reynolds-Zahl Re ist definiert als $Re = w\, d_h / v$, wobei v die kinematische Viskosität, w die mittlere Überstromgeschwindigkeit der Membran und $d_h$ den hydraulischen Durchmesser als charakteristische Länge des Membranmoduls beschreibt.

Die Nanofiltrationseinrichtung kann eine Vielzahl von Trennstufen in Form (seriell oder parallel geschalteter) Membranmodule umfassen, wobei nach jeder Trennstufe das jeweilige erhaltene Permeat als Zulauf der nächsten Trennstufe zugeführt wird, und das Permeat aus der letzten Trennstufe der thermischen Trennung zugeführt wird. Jede Trennstufe umfasst mindestens ein Membranmodul, wobei jedes Membranmodul eine einzelne oder mehrere Nanofiltrationsmembranen umfasst. Unter einem Membranmodul versteht der Fachmann eine praktisch handhabbare anwendungsspezifische Anordnung der Membran in einer Baugruppe.

Der im erfindungsgemäßen Verfahren auf den Schritt des Trennens in einer Nanofiltrationsanlage folgende Schritt der thermischen Trennung des Permeats umfasst typischerweise eine Destillation oder Dünnschichtverdampfer oder Fallfilmverdampfer oder deren Kombination, bei der die erste Fraktion als Kopfprodukt erhalten wird und die zweite Fraktion als Sumpfprodukt. Vorzugsweise ist nach dem Schritt der thermischen Trennung eine weitere Trennung der zweiten Fraktion der thermischen Trennung mittels einer Nanofiltrationseinrichtung nicht erforderlich. Eine weitere Trennung der als Sumpfprodukt erhaltenen zweiten Fraktion ist insbesondere dann entbehrlich, wenn bereits bei der vorausgehenden Trennung des bei der Hydroformylierung resultierenden Produktgemisches in der Nanofiltrationseinrichtung der Übergangsmetallkomplex-Katalysator in so hohem Maße im Retentat zurückgehalten wird, dass im Permeat nur eine so geringe Menge an Katalysator vorhanden ist, dass der Aufwand einer Rückgewinnung aus der hochsiedenden (zweiten) Fraktion der thermischen Trennung sich wirtschaftlich nicht lohnt.

Um die oben genannte Aufgabe zu lösen, ist es zudem vorteilhaft, die technische Hydroformylierung so zu führen, dass die Folgereaktionen, welche zur Bildung von 3-Methylbutansäure und Hochsiedern führen, möglichst nur in geringem Maße ablaufen. Dies lässt sich durch geeignete Einstellung eines oder mehrerer Verfahrensparameter erreichen, insbesondere aus der Gruppe bestehend aus Druck, Temperatur, mittlere Verweilzeit des Reaktionsgemischs im Hydroformylierungsreaktor, Zusammensetzung des Synthesegases, Konzentration des Übergangsmetalls und Übergangsmetall-Ligand-Verhältnis des Übergangsmetallkomplex-Katalysators.

Unter Reaktionsgemisch wird dabei das gesamte im Hydroformylierungsreaktor vorliegende Gemisch umfassend die Edukte (Isobuten und Synthesegas), das primäre Produkt der Hydroformylierung (3-Methylbutanal), daraus gebildete Folgeprodukte (3-Methylbutansäure und Hochsieder) sowie den Übergangsmetallkomplex-Katalysator verstanden. Aus diesem Reaktionsgemisch wird durch Hydroformylierung des Edukts Isobuten, gegebenenfalls nach Abtrennung nicht umgesetzten Isobutens, das resultierende und durch Nanofiltration zu trennende Produktgemisch erhalten, umfassend zumindest 3-Methylbutanal, Folgeprodukte in Form von Hochsiedern und 3-Methylbutansäure und den

Übergangsmetallkomplex-Katalysator und dessen freie Liganden. Sofern nicht umgesetzte Edukte (Isobuten, Wasserstoff, Kohlenmonoxid) nicht vor der Nanofiltrationseinrichtung aus dem Reaktoraustrag abgeschieden werden, sind diese Teil des in den Feed der Nanofiltrationseinrichtung gefahrenen Produktgemisches. Grundsätzlich ist ein vollständiger Umsatz des Isobutens gewünscht, lässt sich aber in der industriellen Praxis nicht immer erreichen. Übliche Umsatzraten liegen über 95 Gew%. Nicht umgesetztes Isobuten wird entweder unmittelbar vor der Nanofiltrationseinrichung abgetrennt, damit ihr Feed nahezu Isobuten-frei ist. Alternativ ist es möglich, verbleibendes Isobuten durch die Membranen permeieren zu lassen und es im Rahmen der ohnehin erforderlichen thermischen Abtrennung des Zielprodukts 3MBA zu entfernen und in den Hydrofomylierungsreaktor zurückzufahren. Diese Variante ist bevorzugt, da thermische Trennverfahren den Katalysatorkomplex schädigen. Das Edukt Kohlenmonoxid sollte indes vorzugsweise auch im Feed, Permeat und Retentat der Nanofiltrationseinrichtung enthalten sein, um den Katalysatorkomplex zu stabilisieren.

In einer vorteilhaften Weiterentwicklung des erfindungsgemäßen Verfahrens werden bei dem Schritt des Hydroformylierens des isobutenhaltigen Kohlenwasserstoffstroms ein oder mehrere Verfahrensparameter so eingestellt, dass die Gesamtkonzentration an Folgeprodukten in Form von Hochsiedern und 3-Methylbutansäure, gewichtsmäßig bezogen auf das Produktgemisch, also den Reaktoraustrag, 30 Gewichts-% oder kleiner ist, wobei der oder die einzustellenden Parameter bevorzugt ausgewählt sind aus der Gruppe bestehend aus Druck, Temperatur, mittlere Verweilzeit des Reaktionsgemischs im Hydroformylierungsreaktor, Zusammensetzung des Synthesegases, Konzentration des Übergangsmetalls und Übergangsmetall-Ligand-Verhältnis des Übergangsmetallkomplex-Katalysators.

Durch eine Verminderung der mittleren Verweilzeit des Reaktionsgemisches im Hydroformylierungsreaktor lässt sich die für die unerwünschten Folgereaktionen zur Verfügung stehende Zeit vermindern, wobei die mittlere Verweilzeit jedoch andererseits ausreichend hoch sein muss, um einen wirtschaftlich sinnvollen Umsetzungsgrad der Edukte zu erlauben. Die mittlere Verweilzeit kann beispielsweise durch die Auslegung der Länge des Reaktors beeinflusst werden.

Durch eine Verminderung der Temperatur im Hydroformylierungsreaktor lässt sich die Geschwindigkeit der unerwünschten Folgereaktionen vermindern, wobei die Temperatur jedoch andererseits ausreichend hoch sein muss, um einen wirtschaftlich sinnvollen Umsetzungsgrad der Edukte zu erlauben.

Bevorzugt werden bei dem Schritt des Hydroformylierens des isobutenhaltigen Kohlenwasserstoffstroms ein oder mehrere Verfahrensparameter, insbesondere die Temperatur und/oder die mittlere Verweilzeit so eingestellt, dass die Konzentration der 3-Methylbutansäure im der Nanofiltrationseinrichtung zuzuführenden Produktgemisch im Bereich zwischen 0.004 und 0.1 Gew-% liegt, vorzugsweise im Bereich zwischen 0.004 und 0.03 Gew-%.

Die Konzentration des Katalysatorkomplexes im resultierenden Permeat beträgt bevorzugt 0.03 Gew-% oder weniger.

Erfindungsgemäß wird der Schritt des Hydroformylierens des isobutenhaltigen Kohlenwasserstoffstroms bevorzugt

- bei einem Druck im Bereich von 0,2 bis 8,0 MPa,
  und/oder
- bei einer Temperatur im Bereich von 70 bis 130°C
  und/oder
- mit einer mittleren Verweilzeit des Reaktionsgemischs im Hydroformylierungsreaktor im Bereich von 1 bis 4 Stunden

und/oder

- einer Synthesegaszusammensetzung ($CO:H_2$) von 1:3 bis 3:1

und/oder

- einer Übergangsmetallkonzentration im Bereich von 10 bis 100 ppm im Hydroformylierungsreaktor

und/oder

- einem Übergangsmetall-Ligand-Verhältnis im Bereich von 1:4 bis 1:50

durchgeführt.

Im Hydroformylierungsschritt des erfindungsgemäßen Verfahrens wird bevorzugt ein Übergangsmetallkomplex-Katalysator eingesetzt, dessen Übergangsmetall Rhodium ist und/oder dessen Ligand bzw. dessen Liganden ausgewählt sind aus der Gruppe der Organophosphor-Liganden. Möglich ist auch der Einsatz eines Übergangsmetallkomplex-Katalysators, dessen Übergangsmetall Cobalt ist.

In eigenen Untersuchungen wurde festgestellt, dass bei Temperaturen ab 130°C im Hydroformylierungsreaktor

die Bildung von 3-Methylbutansäure und daraus entstehenden Hochsiedern deutlich zunimmt. Daher ist es erfindungsgemäß bevorzugt, dass die Temperatur im Hydroformylierungsreaktor 130°C nicht überschreitet. Bevorzugt wird die Hydroformylierung bei einer Temperatur im Bereich von 80 bis 110 °C durchgeführt. Unabhängig davon können einzelne oder alle weiteren oben genannten Verfahrensparameter dahingehend optimiert werden, dass die Bildung von 3-Methylbutansäure und Hochsiedern begrenzt wird.

Bevorzugt werden in einem erfindungsgemäßen Verfahren zum Herstellen eines Produktgemisches durch technische Hydroformylierung eines Isobuten enthaltenden Kohlenwasserstoffstroms und zum Auftrennen des erhaltenen Produktgemisches einzelne oder alle Merkmale der oben als besonders bevorzugt hervorgehobenen Varianten miteinander kombiniert, um eine optimale Abtrennung der 3-Methylbutansäure vom in den Reaktor zurückzuführenden Übergangsmetallkomplex-Katalysator, verbunden mit einer geringen Bildung von 3-Methylbutansäure und Hochsiedern bei der Hydroformylierung, zu erreichen. Daher umfasst ein bevorzugtes erfindungsgemäßes Verfahren die Schritte

- Hydroformylieren eines isobutenhaltigen Kohlenwasserstoffstroms in Gegenwart eines Rhodiumkomplex-Katalysators mit einem oder mehreren Organophosphorliganden bei einem Druck im Bereich von 0.2 bis 8 MPa und einer Temperatur im Bereich von 70 bis 130 °C mit einer mittleren Verweilzeit im Bereich von 1 bis 4 h einer Synthesegaszusammensetzung $CO:H_2$ von 1:3 bis 3:1, einer Rhodiumkonzentration im Reaktor im Bereich von 10 bis 100ppm und einem Rhodium-Ligand-Verhältnis im Bereich von 1:4 bis 1:50;
- Trennen des resultierenden Produktgemisches der Hydroformylierung mittels einer Nanofiltrationseinrichtung bei einer Temperatur im Bereich von 10 bis 150 °C, einem transmembranen Druck im Bereich von 0.5 bis 6 MPa, bei einer Reynoldszahl zwischen 170 und 900, und einem Kohlenmonoxid-Partialdruck von größer 0.2 MPa in Zulauf, Retentat und Permeat jeder Membrantrennstufe;
- thermisches Trennen des resultierenden Permeats der Nanofiltrationseinrichtung mittels Destillation in eine erste Fraktion und eine zweite Fraktion, wobei die erste Fraktion eine höhere Konzentration an 3-Methylbutanal als die zweite Fraktion und eine geringere Konzentration an Hochsiedern als die zweite Fraktion enthält,
- Rückführen zumindest eines Teilstroms des resultierenden Retentats der Nanofiltrationseinrichtung in den Hydroformylierungsreaktor.

Um die oben genannte Aufgabe zu lösen, ist es notwendig, die Konzentration der gebildeten 3-Methylbutansäure zu überwachen, und bei Überschreiten festgelegter Höchstwerte geeignete Gegenmaßnahmen zu ergreifen.

Geeignete Methoden zur Überwachung der Konzentration der 3-Methylbutansäure im Retentat der Nanofiltrationseinrichtung, und vorzugsweise auch im resultierenden Produktgemisch der Hydroformylierung und/oder im Zulauf und/oder im Permeat der Nanofiltrationseinrichtung umfassen eine Messung der Konzentration der 3-Methylbutansäure in dem jeweiligen Prozessstrom mit einer Messmethode ausgewählt aus der Gruppe bestehend aus Gaschromatographie. Durch Überwachen der Konzentration der 3-Methylbutansäure im Retentat der Nanofiltrationseinrichtung lässt sich kontrollieren, wie hoch die Menge an 3-Methylbutansäure ist, die mit dem im Retentat der Nanofiltration angereicherten Übergangsmetallkomplex-Katalysator in den Hydroformylierungsreaktor zurückgeführt wird. Ein Anstieg der Konzentration der 3-Methylbutansäure im Retentat der Nanofiltrationseinrichtung könnte auf eine Blockade der Nanofiltrationsmembran durch Ablagerungen (Membrane fouling) hinweisen. In einer bevorzugten Variante des erfindungsgemäßen Verfahrens wird beim Überschreiten eines festgelegten Höchstwerts der Konzentration der 3-Methylbutansäure im Retentat der Nanofiltrationseinrichtung das Rückführen des Retentats in den Hydroformylierungsreaktor unterbrochen. Dadurch lässt sich verhindern, dass im Hydroformylierungsreaktor die Konzentration an 3-Methylbutansäure ansteigt, und so die unerwünschte Bildung von Hochsiedern begünstigt wird.

In einer besonders bevorzugten Variante des erfindungsgemäßen Verfahrens wird beim Überschreiten eines festgelegten Höchstwerts der Konzentration der 3-Methylbutansäure im Retentat der Nanofiltrationseinrichtung zusätzlich eine Kontrolle der Funktion der Nanofiltrationseinrichtung und, falls erforderlich, ein Austausch oder eine Regeneration einer oder mehrerer Nanofiltrationsmembranen veranlasst. Eine solche Maßnahme könnte insbesondere dann angezeigt sein, wenn gleichzeitig die ebenfalls überwachte Konzentration der 3-Methylbutansäure im Permeat unter einen bestimmten Mindestwert absinkt (siehe weiter unten).

Durch Überwachen der Konzentration der 3-Methylbutansäure im Zulauf der Nanofiltrationseinrichtung bzw. im resultierenden Produktgemisch der Hydroformylierung lässt sich kontrollieren, wie hoch die Menge an 3-Methylbutansäure in dem durch Hydroformylieren des isobutenhaltigen Kohlenwasserstoffstroms erhaltenen Produktgemisch ist, das - vorzugsweise nach Entspannung unter Abtrennung nicht umgesetzten Isobutens und optional noch Vorfiltration - der Nanofiltrationseinrichtung als Zulauf zugeführt wird. Eine übermäßig hohe Konzentration an 3-Methylbutansäure im Zulauf der Nanofiltrationseinrichtung ist ein Hinweis auf bezüglich der Vermeidung der Bildung von Hochsiedern nicht optimal eingestellte Verfahrensparameter der technischen Hydroformylierung im Hydroformylierungsreaktor. Ein plötzliches Ansteigen der Konzentration der 3-Methylbutansäure im Zulauf der Nanofiltrationseinrichtung könnte sogar ein Hinweis auf ein Versagen oder Außer-Kontrolle-Geraten der Regelung eines oder mehrerer Verfahrensparameter der Hydroformylierung sein. Somit kann diese beschriebene Variante des erfindungsgemäßen Verfahrens zur sicheren

Kontrolle der Verfahrensparameter der Hydroformylierung beitragen, und gegebenenfalls sogar zur Vermeidung von Fehlproduktionen und Betriebsstörungen.

In einer bevorzugten Variante des erfindungsgemäßen Verfahrens werden beim Überschreiten eines festgelegten Höchstwerts der Konzentration der 3-Methylbutansäure im Zulauf der Nanofiltrationseinrichtung ein oder mehrere Verfahrensparameter des Schrittes des Hydroformylierens des isobutenhaltigen Kohlenwasserstoffstroms im Hydroformylierungsreaktor so modifiziert, dass die Konzentration der 3-Methylbutansäure im Zulauf der Nanofiltrationseinrichtung auf oder unter den festgelegten Höchstwert abgesenkt wird. Vorzugsweise erfolgt die geeignete Einstellung eines oder mehrerer Verfahrensparameter der Hydroformylierung wie oben beschrieben, wobei die einzustellenden Parameter insbesondere ausgewählt sind aus der Gruppe bestehend aus Druck, Temperatur, mittlere Verweilzeit des Reaktionsgemischs im Hydroformylierungsreaktor, Zusammensetzung des Synthesegases, Konzentration des Übergangsmetalls und Übergangsmetall-Ligand-Verhältnis des Übergangsmetallkomplex-Katalysators.

Durch Überwachen der Konzentration der 3-Methylbutansäure im Permeat der Nanofiltrationseinrichtung lässt sich kontrollieren, wie hoch die Menge an 3-Methylbutansäure ist, die durch die eine oder mehreren Nanofiltrationsmembranen der Nanofiltrationsanlage permeiert. Dadurch lässt sich der ordnungsgemäße Betrieb der Nanofiltrationseinrichtung überwachen. Ein Abfall der Konzentration der 3-Methylbutansäure im Permeat der Nanofiltrationseinrichtung könnte auf eine Blockade der Nanofiltrationsmembran durch Ablagerungen (Membranfouling) hinweisen. In einer bevorzugten Variante des erfindungsgemäßen Verfahrens wird daher beim Unterschreiten eines festgelegten Mindestwerts der Konzentration der 3-Methylbutansäure im Permeat der Nanofiltrationseinrichtung eine Kontrolle der Funktion der Nanofiltrationseinrichtung und, falls erforderlich, ein Austausch oder eine Regeneration einer oder mehrere Nanofiltrationsmembranen veranlasst.

Bevorzugt werden in einem erfindungsgemäßen Verfahren zum Herstellen eines Produktgemisches durch technische Hydroformylierung eines Isobuten enthaltenden Kohlenwasserstoffstroms und zum Auftrennen des erhaltenen Produktgemisches einzelne oder alle Merkmale der oben als besonders bevorzugt hervorgehobenen Weiterentwicklungen und Varianten miteinander kombiniert, um eine optimale Abtrennung der 3-Methylbutansäure vom in den Reaktor zurückzuführenden Übergangsmetallkomplex-Katalysator sowie eine geringe Bildung von 3-Methylbutansäure und Hochsiedern bei der Hydroformylierung und eine umfassende Überwachung der Konzentration der 3-Methylbutansäure in allen relevanten Stoffströmen des erfindungsgemäßen Verfahrens zu erreichen.

Die vorliegende Offenbarung betrifft zudem eine Vorrichtung für die Durchführung des erfindungsgemäßen Verfahrens, insbesondere für die oben beschriebenen bevorzugten Varianten des erfindungsgemäßen Verfahrens. Eine Vorrichtung zur Durchführung des erfindungsgemäßen Verfahrens umfasst:

- einen Hydroformylierungsreaktor (R) zum Hydroformylieren eines isobutenhaltigen Kohlenwasserstoffstroms (1) in Gegenwart eines Übergangsmetallkomplex-Katalysators,
- eine Nanofiltrationseinrichtung (M) umfassend eine oder mehrere Membrantrennstufen zum Trennen des im Hydroformylierungsreaktor (R) gebildeten Produktgemisches (2), so dass im resultierenden Retentat (3) der Nanofiltrationseinrichtung (M) der Übergangsmetallkomplex-Katalysator und dessen freie Liganden gegenüber dem 3-Methylbutanal und der 3-Methylbutansäure angereichert sind, und im resultierenden Permeat der Nanofiltrationseinrichtung (M) 3-Methylbutanal und 3-Methylbutansäure gegenüber dem Übergangsmetallkomplex-Katalysator angereichert sind, dergestalt, dass die Konzentration der der 3-Methylbutansäure im resultierenden Retentat (3) geringer ist als im resultierenden Permeat (4), wobei die Nanofiltrationseinrichtung (M) eine oder mehrere Nanofiltrationsmembranen umfasst, wobei die oder mindestens eine der Nanofiltrationsmembranen einen Rückhalt für 3-Methylbutansäure von -1 oder kleiner, besonders bevorzugt -5 oder kleiner, und insbesondere -10 oder kleiner aufweist,
- mindestens eine thermische Trenneinrichtung (D) zum thermischen Trennen des resultierenden Permeats (4) der Nanofiltrationseinrichtung (M) in eine erste Fraktion (5) und eine zweite Fraktion (6), wobei die erste Fraktion (5) eine höhere Konzentration an 3-Methylbutanal als die zweite Fraktion (6) und eine geringere Konzentration an Folgeprodukten in Form von Hochsiedern und 3-Methylbutansäure als die zweite Fraktion (6) enthält,
- Mittel zum Rückführen zumindest eines Teilstroms des resultierenden Retentats (3) der Nanofiltrationseinrichtung (M) in den Hydroformylierungsreaktor (R).

Die Vorrichtung zur Durchführung des erfindungsgemäßen Verfahrens kann außerdem im Stand der Technik übliche Bestandteile enthalten, z.B. Pumpen, übliche Dosier-, Mess-, Steuer- und Regelungseinrichtungen, Heiz- und Kühlvorrichtungen etc. Derartige zum Stand der Technik gehörende und in der chemischen Verfahrenstechnik übliche Bestandteile von Vorrichtungen für technische Synthese- und Trennverfahren sind dem Fachmann bekannt.

Als Hydroformylierungsreaktor wird bevorzugt ein Apparat eingesetzt aus der Gruppe bestehend aus Rührkessel, Blasensäule, Strahldüsenreaktor, Rohrreaktor und Schlaufenreaktor, wobei der Apparat mit Einbauten versehen sein kann.

Der Hydroformylierungsreaktor ist in einer besonders bevorzugten Ausführungsform als Blasensäulenreaktor

ausgebildet. In dieser erfindungsgemäßen Vorrichtung ist die Längenausdehnung des Hydroformylierungsreaktors bevorzugt so gewählt, dass eine Verweilzeit des Reaktionsgemisches im Hydroformylierungsreaktor resultiert, die zwar ausreichend ist für einen wirtschaftlich sinnvollen Grad der Umsetzung der Edukte zu dem primären Hydroformylierungsprodukt 3-Methylbutanal, jedoch keinen hohen Grad der Umsetzung des primären Hydroformylierungsprodukts 3-Methylbutanal zu Folgeprodukten in Form von 3-Methylbutansäure und Hochsiedern erlaubt.

Die Nanofiltrationseinrichtung der Vorrichtung umfasst vorzugsweise ein oder mehrere Membranmodule. In diesen Modulen werden die Nanofiltrationsmembranen so angeordnet, dass die Retentatseite der Nanofiltrationsmembran derart überströmt werden kann, dass der Konzentrationspolarisation der abgetrennten Komponenten, d. h. des Übergangsmetallkomplex-Katalysators, entgegengewirkt und außerdem die nötige treibende Kraft (Druck) aufgeprägt werden kann. Das Permeat wird im Permeatsammelraum auf der Permeatseite der Nanofiltrationsmembranen zusammengeführt und aus dem Modul abgeführt. Gängige Membranmodule weisen die Nanofiltrationsmembranen in Form von Membranscheiben, Membrankissen oder Membrantaschen auf. Besonders bevorzugt sind Membranmodule mit offenkanaligen Kissenmodulsystemen, bei denen die Nanofiltrationsmembranen zu Membrantaschen oder - kissen thermisch verschweißt oder verklebt sind, oder Wickelmodule, bei denen die Nanofiltrationsmembranen zu Membrantaschen oder Membrankissen verklebt oder verschweißt sind und mit Feed-spacern um ein Permeatsammelrohr aufgewickelt sind.

Bevorzugt ist die Nanofiltrationseinrichtung der Vorrichtung so ausgestaltet, dass sich die oben beschriebenen bevorzugten Verfahrensparameter, insbesondere Verfahrensparameter aus der Gruppe bestehend aus Druck, Temperatur, mittlere Verweilzeit des Reaktionsgemischs im Hydroformylierungsreaktor, Zusammensetzung des Synthesegases, Konzentration des Übergangsmetalls und Übergangsmetall-Ligand-Verhältnis des Übergangsmetallkomplex-Katalysators einstellen lassen und/oder die oben beschriebenen bevorzugten Varianten der Ausführung des Schritts der Trennung des resultierenden Produktgemischs der Hydroformylierung realisieren lassen.

Die Nanofiltrationseinrichtung kann eine Vielzahl von Trennstufen umfassen, die so angeordnet sind, dass nach jeder Trennstufe das erhaltene Permeat jeweils als Zulauf der nächsten Trennstufe zugeführt wird, und das Permeat aus der letzten Trennstufe der thermischen Trennung zugeführt wird. Jede Trennstufe kann als ein Membranmodul ausgelegt sein oder mehrere parallel angeordnete Membranmodule umfassen. Jedes Membranmodul kann eine oder mehrere parallel angeordnete Nanofiltrationsmembranen umfassen.

Hinsichtlich der Auswahl einer oder mehrerer geeigneter Nanofiltrationsmembranen für die Nanofiltrationseinrichtung der Vorrichtung gelten die obigen Ausführungen zur Auswahl einer oder mehrerer geeigneter Nanofiltrationsmembranen für das erfindungsgemäße Verfahren entsprechend.

Bevorzugt umfasst die Nanofiltrationseinrichtung eine oder mehrere Nanofiltrationsmembranen, wobei die oder mindestens eine der Nanofiltrationsmembranen eine Trenngrenze im Bereich von 150 bis 2000 g/mol, bevorzugt 200 bis 600g/mol und besonders bevorzugt von 350 bis 500 g/mol aufweist.

In einer bevorzugten Ausführungsform, die für eine oben beschriebene bevorzugte Variante des erfindungsgemäßen Verfahrens besonders angepasst ist, umfasst die Vorrichtung zusätzlich

- eine Vorrichtung zur Bestimmung der Konzentration von 3-Methylbutansäure im Retentat der Nanofiltrationseinrichtung vorzugsweise zusätzlich eine Vorrichtung zur Bestimmung der Konzentration von 3-Methylbutansäure im resultierenden Produktgemisch der Hydroformylierung und/oder im Zulauf der Nanofiltrationseinrichtung und/oder eine Vorrichtung zur Bestimmung der Konzentration von 3-Methylbutansäure im Permeat der Nanofiltrationseinrichtung,

oder

- eine Vorrichtung zur Entnahme von Proben aus dem Retentat der Nanofiltrationseinrichtung, vorzugsweise zusätzlich eine Vorrichtung zur Entnahme von Proben aus dem resultierenden Produktgemisch der Hydroformylierung und/oder aus dem Zulauf der Nanofiltrationseinrichtung und/oder eine Vorrichtung zur Entnahme von Proben aus dem Permeat der Nanofiltrationseinrichtung.

Die Vorrichtung zur Bestimmung der Konzentration von 3-Methylbutansäure im Zulauf der Nanofiltrationseinrichtung ist vorzugsweise ausgewählt aus der Gruppe bestehend aus Gaschromatographen. Entsprechendes gilt für die optional vorgesehenen Vorrichtungen zur Bestimmung der Konzentration von 3-Methylbutansäure im Retentat und/oder Permeat der Nanofiltrationseinrichtung.

Falls eine direkte Messung der Konzentration von 3-Methylbutansäure im resultierenden Produktgemisch der Hydroformylierung und/oder Zulauf und/oder Retentat und/oder Permeat der Nanofiltrationseinrichtung nicht möglich oder zu aufwändig ist, können Vorrichtungen zur Entnahme von Proben aus dem im resultierenden Produktgemisch der Hydroformylierung, dem Zulauf und/oder Retentat und/oder Permeat der Nanofiltrationseinrichtung vorgesehen werden. In den mit dieser Vorrichtung regelmäßig, d. h. zu bestimmten Zeitpunkten oder in bestimmten Intervallen, bzw.

bei Bedarf aus dem Zulauf, Retentat und/oder Permeat entnommenen Proben wird beispielsweise in einem Prozesskontrolllabor die Konzentration der 3-Methylbutansäure bestimmt.

Hinsichtlich der aus der Überwachung der Konzentration der 3-Methylbutansäure im resultierenden Produktgemisch der Hydroformylierung und/oder Zulauf, im Retentat und im Permeat der Nanofiltrationseinrichtung erhältlichen Informationen und der mit dieser Überwachung erzielbaren Vorteile gelten die obigen Ausführungen betreffend erfindungsgemäßen Verfahrens umfassend den Schritt

- Überwachen der Konzentration der 3-Methylbutansäure im Retentat der Nanofiltrationseinrichtung, und vorzugsweise auch im resultierenden Produktgemisch der Hydroformylierung und/oder im Zulauf und/oder im Permeat der Nanofiltrationseinrichtung.

Die vorliegende Offenbarung umfasst ferner die Verwendung einer Vorrichtung, insbesondere in ihren bevorzugten Ausführungsformen, zum Herstellen eines Produktgemisches durch technische Hydroformylierung eines Isobuten enthaltenden Kohlenwasserstoffstroms und zum Auftrennen des erhaltenen Produktgemisches, insbesondere nach einem Verfahren gemäß einer der bevorzugten Varianten des oben beschriebenen erfindungsgemäßen Verfahrens. Weitere bevorzugte Ausfühungsformen der Erfindung ergeben sich aus den Ansprüchen.

Die Erfindung wird nachfolgend anhand eines Ausführungsbeispiels und der Figuren näher beschrieben, ohne dass diese den Schutzbereich der Patentansprüche beschränken. Es zeigen:

Fig. 1: Vorrichtung zum Herstellen eines Produktgemisches durch technische Hydroformylierung eines Isobuten enthaltenden Kohlenwasserstoffstroms und zum Auftrennen des erhaltenen Produktgemisches nach dem erfindungsgemäßen Verfahren;

Fig. 2: Graphische Darstellung 3MBS-Konzentrationen im Feed, Retentat und Permeat der Nanofiltration;

Fig. 3 Graphische Darstellung des 3MBS-Rückhalt der Nanofiltration.

Zum Herstellen eines Produktgemisches durch technische Hydroformylierung eines Isobuten enthaltenden Kohlenwasserstoffstroms 1 und zum Auftrennen des erhaltenen Produktgemisches 2 wird eine in Figur 1 skizzierte Vorrichtung umfassend einen Hydroformylierungsreaktor R in Form einer Blasensäule, eine Nanofiltrationseinrichtung M und eine Destillationseinrichtung D eingesetzt. In der Vorrichtung sind Probeentnahmevorrichtungen P vorgesehen, die es ermöglichen, in bestimmten Intervallen aus dem den Reaktor R verlassenden Produktstrom 2 sowie dem Retentat 3 und dem Permeat 4 der Nanofiltrationseinrichtung M Proben zu entnehmen.

Die Nanofiltrationseinrichtung M umfasst ein Membranmodul in Form eines Wickelmoduls. Die Nanofiltrationsmembran ist eine Membran des Typs STARMEM 122 der Firma W. R. Grace & Co und enthält Polyimid P84.

Dem Hydroformylierungsreaktor R wird ein Kohlenwasserstoffstrom 1 umfassend Isobuten und Synthesegas zugeführt. Im Reaktor R erfolgt die Hydroformylierung des Isobutens zu 3-Methylbutanal bei einem Druck im Bereich von 0,2 bis 8,0 MPa und einer Temperatur im Bereich von 70 bis 130 °C mit einer mittleren Verweilzeit im Bereich von 1 bis 4 Stunden, einer Synthesegaszusammensetzung (CO/H2) von 1:3 bis 3:1, einer Rhodiumkonzentration im Bereich von 20 bis 100 ppm und einem Rhodium-Ligand-Verhältnis im Bereich von 1:4 bis 1:50.

Das resultierende Produktgemisch 2 der Hydroformylierung, in welchem die Konzentration der 3-Methylbutansäure im Bereich zwischen 0,01 und 0,04 Gew-% liegt, wird anschließend in einer Nanofiltrationseinrichtung M bei einer Temperatur im Bereich von 30 bis 35 °C, einem transmembranen Druck im Bereich von 3,1 bis 3,8 MPa und einem Kohlenmonoxid-Partialdruck im Bereich von 0,9 bis 1,2 MPa getrennt. Die Nanofiltrationseinrichtung M ist so ausgelegt, dass beim Überströmen der Nanofiltrationsmembran die Reynoldszahl zwischen 170 und 900 liegt.

Aus der Nanofiltrationseinrichtung M werden ein resultierendes Retentat 3 und ein resultierendes Permeat 4 entnommen. Das Verhältnis aus der Membran zugeführten Produktgemisch 2 - auch als Feed bezeichnet - und dem Permeat 4 wurde auf 0,82 eingestellt. Der Retentatstrom 3, in welchem der Übergangsmetall-Komplexkatalysator angereichert ist, wird in den Reaktor R zurückgeführt.

Aus dem den Reaktor R verlassenden Produktgemisch 2, dem Retentat 3 und dem Permeat 4 werden in regelmäßigen Intervallen mit Probeentnahmevorrichtungen P Proben entnommen, um die Konzentration an 3-Methylbutansäure in dem jeweiligen Stoffstrom zu bestimmen.

Das resultierende Permeat 4 der Nanofiltrationseinrichtung M, in welchem das primäre Hydroformylierungsprodukt 3-Methylbutanal sowie die 3-Methylbutansäure angereichet sind, wird mittels Destillation in einer thermischen Trenneinrichtung in Gestalt einer Destillationsanlage D in eine erste Fraktion 5 und eine zweite Fraktion 6 getrennt. In der ersten Fraktion 5, die als Kopfprodukt erhalten wird, ist die Konzentration an 3-Methylbutanal höher als in der zweiten Fraktion 6 und die Konzentration an 3-Methylbutansäure und Hochsiedern geringer als in der zweiten Fraktion.

Figur 2 zeigt die 3MBS-Konzentration im Feed, Retentat und Permeat der Nanofiltration M über einem Zeitraum

von 500 Stunden. Figur 3 zeigt deutlich negative Rückhalte der eingesetzten Starmem 122 Membran für die 3-MBS im Nanofiltrationsschritt. Der Rückhalt ist definiert als 1-(Konzentration Permeat)/(Konzentration Retentat).

**Bezugszeichenliste**

| | |
|---|---|
| 1 | Kohlenwasserstoffstrom |
| 2 | Produktgemisch |
| 3 | resultierendes Retentat |
| 4 | resultierendes Permeat |
| 5 | erste Fraktion |
| 6 | zweite Fraktion |
| D | thermische Trenneinrichtung |
| R | Hydroformylierungsreaktor |
| M | Nanofiltrationseinrichtung |
| P | Probennahme |

## Patentansprüche

**1.** Verfahren zum Herstellen eines Produktgemisches (2) durch technische Hydroformylierung eines Isobuten enthaltenden Kohlenwasserstoffstroms (1) und zum Auftrennen des erhaltenen Produktgemisches (2), mit folgenden Schritten:

a) Hydroformylieren des Isobuten enthaltenden Kohlenwasserstoffstroms (1) in einem Hydroformylierungsreaktor (R) in Gegenwart eines Übergangsmetallkomplex-Katalysators, so dass ein Produktgemisch (2) erhalten wird, das zumindest 3-Methylbutanal, Folgeprodukte in Form von Hochsiedern und 3-Methylbutansäure, sowie den Übergangsmetallkomplex-Katalysator nebst dessen freie Liganden umfasst,
b) Trennen des Produktgemisches (2) mittels einer eine oder mehrere Membrantrennstufen umfassenden Nanofiltrationseinrichtung (M), sodass im resultierenden Retentat (3) der Nanofiltrationseinrichtung (M) der Übergangsmetallkomplex-Katalysator und dessen freie Liganden gegenüber 3-Methylbutanal und 3-Methylbutansäure angereichert sind, und sodass im resultierenden Permeat (4) der Nanofiltrationseinrichtung (M) 3-Methylbutanal und 3-Methylbutansäure jeweils gegenüber dem Übergangsmetallkomplex-Katalysator angereichert sind, wobei im resultierendem Retentat (3) die Konzentration der 3-Methylbutansäure geringer ist als im Permeat (4),
c) Trennen des resultierenden Permeats (4) der Nanofiltrationseinrichtung (M) mittels einer eine oder mehrere Trennstufen umfassenden thermischen Trenneinrichtung (D) in zumindest eine erste Fraktion (5) und eine zweite Fraktion (6), wobei die erste Fraktion (5) eine höhere Konzentration an 3-Methylbutanal als die zweite Fraktion (6) und eine geringere Konzentration an Folgeprodukten in Form von Hochsiedern und 3-Methylbutansäure als die zweite Fraktion (6) enthält,
d) Rückführen zumindest eines Teilstroms des resultierenden Retentats (3) der Nanofiltrationseinrichtung (M) in den Hydroformylierungsreaktor (R);
und ferner umfassend den Schritt:

- Überwachen der Konzentration der 3-Methylbutansäure im resultierenden Retentat (3) der Nanofiltrationseinrichtung, und vorzugsweise auch im Produktgemisch (2) und/oder im resultierenden Permeat (4) der Nanofiltrationseinrichtung.

**2.** Verfahren nach Anspruch 1, **dadurch gekennzeichnet, dass** bei dem Schritt des Hydroformylierens des isobutenhaltigen Kohlenwasserstoffstroms ein oder mehrere Verfahrensparameter so eingestellt sind, dass die Gesamtkonzentration an Folgeprodukten in Form von Hochsiedern und 3-Methylbutansäure, gewichtsmäßig bezogen auf das gesamte Produktgemisch, 30 Gew% oder kleiner ist,
wobei der oder die einzustellenden Paramater bevorzugt ausgewählt sind aus der Gruppe bestehend aus Druck, Temperatur, mittlere Verweilzeit, Zusammensetzung des Synthesegases, Konzentration des Übergangsmetalls und Übergangsmetall-Ligand-Verhältnis des Übergangsmetallkomplex-Katalysators.

**3.** Verfahren nach Anspruch 1 oder 2, **dadurch gekennzeichnet, dass** der Schritt des Hydroformylierens des isobutenhaltigen Kohlenwasserstoffstroms,

- bei einem Druck im Bereich von 0.2 bis 8 MPa,

und/oder

- bei einer Temperatur im Bereich von 70 bis 130 °C

und/oder

- mit einer mittleren Verweilzeit im Bereich von 1 bis 4 h

und/oder

- einer Synthesegaszusammensetzung $CO:H_2$ von 1:3 bis 3:1

und/oder

- einer Übergangsmetallkonzentration im Bereich von 10 bis 100 ppm

und/oder

- einem Übergangsmetall-Ligand-Verhältnis im Bereich von 1:4 bis 1:50 durchgeführt wird.

**4.** Verfahren nach einem der vorhergehenden Ansprüche, **dadurch gekennzeichnet dass**

- das Übergangsmetall des Übergangsmetallkomplex-Katalysators Rhodium ist

und/oder

- der oder die Liganden des Übergangsmetallkomplex-Katalysators ausgewählt sind aus der Gruppe der Organophosphor-Liganden.

**5.** Verfahren nach einem der vorangehenden Ansprüche, **dadurch gekennzeichnet, dass** ein Übergangsmetallkomplex-Katalysator ausgewählt aus der Gruppe bestehend aus Rhodium und Cobalt eingesetzt wird.

**6.** Verfahren nach einem der vorangehenden Ansprüche, **dadurch gekennzeichnet, dass** in der Nanofiltrationseinrichtung eine oder mehrere Nanofiltrationsmembranen eingesetzt werden, wobei die oder mindestens eine der Nanofiltrationsmembranen einem Rückhalt für 3-Methylbutansäure von -1 oder kleiner, besonders bevorzugt -5 oder kleiner, und insbesondere -10 oder kleiner aufweist.

**7.** Verfahren nach einem der vorhergehenden Ansprüche, **dadurch gekennzeichnet, dass** in der Nanofiltrationseinrichtung eine oder mehrere Nanofiltrationsmembranen umfassend oder bestehend aus einem oder mehreren Imidgruppen enthaltenden Polymeren eingesetzt werden, wobei das oder die Imidgruppen enthaltenden Polymere so ausgewählt sind, das der Rückhalt der Nanofiltrationsmembran für 3-Methylbutansäure -1 oder kleiner, besonders bevorzugt -5 oder kleiner, und insbesondere -10 oder kleiner ist.

**8.** Verfahren nach einem der vorangehenden Ansprüche, **dadurch gekennzeichnet, dass** in der Nanofiltrationseinrichtung eine oder mehrere Nanofiltrationsmembranen eingesetzt werden, wobei die oder mindestens eine der Nanofiltrationsmembranen einer Trenngrenze im Bereich von 150 bis 2000 g/mol, bevorzugt 200 bis 600 g/mol und ganz besonders bevorzugt 350 bis 500 g/mol aufweist.

**9.** Verfahren nach einem der vorhergehenden Ansprüche, **dadurch gekennzeichnet, dass** in der Nanofiltrationseinrichtung eine oder mehrere Nanofiltrationsmembranen umfassend oder bestehend aus einem oder mehreren Imidgruppen enthaltenden Polymeren eingesetzt werden, wobei das oder die Imidgruppen enthaltenden Polymere so ausgewählt sind, dass die Trenngrenze der Nanofiltrationsmembran im Bereich von 150 bis 2000 g/mol liegt.

**10.** Verfahren nach einem der vorangehenden Ansprüche, **dadurch gekennzeichnet, dass** der Schritt des Trennens

des durch Hydroformylieren des isobutenhaltigen Kohlenwasserstoffstroms erhaltenen Produktgemisches in der Nanofiltrationseinrichtung

- bei einer Temperatur im Bereich von 10 bis 150 °C

und/oder

- bei einem transmembranen Druck im Bereich von 0,5 bis 6 MPa

und/oder

- bei einer Reynoldszahl zwischen 55 und 13500, bevorzugt zwischen 100 und 3500 und ganz besonders bevorzugt zwischen 170 und 900,

und/oder

- in Gegenwart von Kohlenmonoxid und/oder Wasserstoff, bevorzugt bei einem Kohlenmonoxid-Partialdruck von mindestens 200 kPa im Zulauf, im Retentat und im Permeat jeder Membrantrennstufe

durchgeführt wird.

**11.** Verfahren nach einem der vorangehenden Ansprüche, **dadurch gekennzeichnet, dass** der Schritt der thermischen Trennung eine Destillation umfasst, bei der die erste Fraktion als Kopfprodukt erhalten wird und die zweite Fraktion als Sumpfprodukt.

**12.** Verfahren nach einem der vorangehenden Ansprüche umfassend die Schritte:

- Hydroformylieren eines isobutenhaltigen Kohlenwasserstoffstroms in Gegenwart eines Rhodiumkomplex-Katalysators mit einem oder mehreren Organophosphorliganden bei einem Druck im Bereich von 0.2 bis 8 MPa und einer Temperatur im Bereich von 70 bis 130 °C mit einer mittleren Verweilzeit im Bereich von 1 bis 4 h einer Synthesegaszusammensetzung $CO:H_2$ von 1:3 bis 3:1, einer Rhodiumkonzentration im Reaktor im Bereich von 10 bis 100ppm und einem Rhodium-Ligand-Verhältnis im Bereich von 1:4 bis 1:50;
- Trennen des resultierenden Produktgemisches der Hydroformylierung mittels einer Nanofiltrationseinrichtung bei einer Temperatur im Bereich von 10 bis 150 °C, einem transmembranen Druck im Bereich von 0.5 bis 6 MPa, bei einer Reynoldszahl zwischen 170 und 900, und einem Kohlenmonoxid-Partialdruck von größer 0.2 MPa in Zulauf, Retentat und Permeat jeder Membrantrennstufe;
- thermisches Trennen des resultierenden Permeats der Nanofiltrationseinrichtung mittels Destillation in eine erste Fraktion und eine zweite Fraktion, wobei die erste Fraktion eine höhere Konzentration an 3-Methylbutanal als die zweite Fraktion und eine geringere Konzentration an Hochsiedern als die zweite Fraktion enthält,
- Rückführen zumindest eines Teilstroms des resultierenden Retentats der Nanofiltrationseinrichtung in den Hydroformylierungsreaktor.

**13.** Verfahren nach einem der vorhergehenden Ansprüche, **dadurch gekennzeichnet, dass** beim Überschreiten eines festgelegten Höchstwerts der Konzentration der 3-Methylbutansäure im resultierenden Retentat der Nanofiltrationseinrichtung das Rückführen des resultierendem Retentats in den Hydroformylierungsreaktor unterbrochen wird.

**14.** Verfahren nach einem der vorhergehenden Ansprüche, **dadurch gekennzeichnet, dass** beim Überschreiten eines festgelegten Höchstwerts der Konzentration der 3-Methylbutansäure im Zulauf der Nanofiltrationseinrichtung ein oder mehrere Verfahrensparameter des Schrittes des Hydroformylieren des isobutenhaltigen Kohlenwasserstoffstroms im Hydroformylierungsreaktor so modifiziert werden, dass die Konzentration der 3-Methylbutansäure im Zulauf der Nanofiltrationseinrichtung unter den festgelegten Höchstwert abgesenkt wird.

## Claims

**1.** Process for preparing a product mixture (2) by industrial hydroformylation of an isobutene-containing hydrocarbon stream (1) and for separating the resulting product mixture (2), comprising the following steps:

a) hydroformylating the isobutene-containing hydrocarbon stream (1) in a hydroformylation reactor (R) in the presence of a transition metal complex catalyst, so as to obtain a product mixture (2) comprising at least 3-methylbutanal, conversion products in the form of high boilers and 3-methylbutanoic acid, and the transition metal complex catalyst along with the free ligands thereof,
b) separating the product mixture (2) by means of a nanofiltration device (M) comprising one or more membrane separation stages, such that the transition metal complex catalyst and the free ligands thereof are enriched in the resulting retentate (3) of the nanofiltration device (M) with respect to 3-methylbutanal and 3-methylbutanoic acid, and such that 3-methylbutanal and 3-methylbutanoic acid are each enriched in the resulting permeate (4) of the nanofiltration device (M) with respect to the transition metal complex catalyst, the concentration of the 3-methylbutanoic acid being lower in the resulting retentate (3) than in the permeate (4),
c) separating the resulting permeate (4) of the nanofiltration device (M) by means of a thermal separating device (D) comprising one or more separation stages into at least one first fraction (5) and a second fraction (6), the first fraction (5) having a higher concentration of 3-methylbutanal than the second fraction (6) and a lower concentration of conversion products in the form of high boilers and 3-methylbutanoic acid than the second fraction (6),
d) recycling at least a substream of the resulting retentate (3) of the nanofiltration device (M) into the hydroformylation reactor (R);

and further comprising the step of:

- monitoring the concentration of 3-methylbutanoic acid in the resulting retentate (3) of the nanofiltration device, and preferably also in the product mixture (2) and/or in the resulting permeate (4) of the nanofiltration device.

**2.** Process according to Claim 1, **characterized in that** one or more process parameters in the step of hydroformylating the isobutenic hydrocarbon stream are set such that the total concentration of conversion products in the form of high boilers and 3-methylbutanoic acid, based on the weight of the overall product mixture, is 30% by weight or less, the parameter(s) to be set preferably being selected from the group consisting of pressure, temperature, mean residence time, composition of the synthesis gas, concentration of the transition metal and transition metal-ligand ratio of the transition metal complex catalyst.

**3.** Process according to Claim 1 or 2, **characterized in that** the step of hydroformylating the isobutenic hydrocarbon stream is performed

- at a pressure in the range from 0.2 to 8 MPa,

and/or

- at a temperature in the range from 70 to 130°C

and/or

- with a mean residence time in the range from 1 to 4 h

and/or

- a synthesis gas composition $CO:H_2$ of 1:3 to 3:1

and/or

- a transition metal concentration in the range from 10 to 100 ppm

and/or

- a transition metal/ligand ratio in the range from 1:4 to 1:50.

**4.** Process according to any of the preceding claims, **characterized in that**

- the transition metal of the transition metal complex catalyst is rhodium

and/or

- the ligand(s) of the transition metal complex catalyst is/are selected from the group of the organophosphorus ligands.

**5.** Process according to any of the preceding claims, **characterized in that** a transition metal complex catalyst selected from the group consisting of rhodium and cobalt is used.

**6.** Process according to any of the preceding claims, **characterized in that** one or more nanofiltration membranes are used in the nanofiltration device, the or at least one of the nanofiltration membranes having a retention for 3-methylbutanoic acid of -1 or less, more preferably -5 or less and especially -10 or less.

**7.** Process according to any of the preceding claims, **characterized in that** one or more nanofiltration membranes which are used in the nanofiltration device comprise or consist of one or more polymers containing imide groups, the polymer(s) containing imide groups being selected such that the retention of the nanofiltration membrane for 3-methylbutanoic acid is -1 or less, more preferably -5 or less, and especially -10 or less.

**8.** Process according to any of the preceding claims, **characterized in that** one or more nanofiltration membranes are used in the nanofiltration device, the or at least one of the nanofiltration membranes having a separation limit in the range from 150 to 2000 g/mol, preferably 200 to 600 g/mol and most preferably 350 to 500 g/mol.

**9.** Process according to any of the preceding claims, **characterized in that** one or more nanofiltration membranes which are used in the nanofiltration device comprise or consist of one or more polymers containing imide groups, the polymer(s) containing imide groups being selected such that the separation limit of the nanofiltration membrane is in the range from 150 to 2000 g/mol.

**10.** Process according to any of the preceding claims, **characterized in that** the step of separating the product mixture obtained by hydroformylating the isobutenic hydrocarbon stream in the nanofiltration device is performed

- at a temperature in the range from 10 to 150°C

and/or

- at a transmembrane pressure in the range from 0.5 to 6 MPa

and/or

- at a Reynolds number between 55 and 13 500, preferably between 100 and 3500 and most preferably between 170 and 900,

and/or

- in the presence of carbon monoxide and/or hydrogen, preferably at a partial carbon monoxide pressure of at least 200 kPa in the feed, in the retentate and in the permeate of each membrane separation stage.

**11.** Process according to any of the preceding claims, **characterized in that** the step of thermal separation comprises a distillation in which the first fraction is obtained as the top product and the second fraction as the bottom product.

**12.** Process according to any of the preceding claims, comprising the steps of:

- hydroformylating an isobutenic hydrocarbon stream in the presence of a rhodium complex catalyst having one or more organophosphorus ligands at a pressure in the range from 0.2 to 8 MPa and a temperature in the range from 70 to 130°C with a mean residence time in the range from 1 to 4 h, a synthesis gas composition $CO:H_2$ of 1:3 to 3:1, a rhodium concentration in the reactor in the range from 10 to 100 ppm and a rhodium/ligand ratio in the range from 1:4 to 1:50;
- separating the resulting product mixture of the hydroformylation by means of a nanofiltration device at a temperature in the range from 10 to 150°C, a transmembrane pressure in the range from 0.5 to 6 MPa, at a Reynolds number between 170 and 900, and a partial carbon monoxide pressure of greater than 0.2 MPa into

feed, retentate and permeate of each membrane separation stage;
- thermally separating the resulting permeate of the nanofiltration device by means of distillation into a first fraction and a second fraction, the first fraction containing a higher concentration of 3-methylbutanal than the second fraction and a lower concentration of high boilers than the second fraction,
- recycling at least a substream of the resulting retentate of the nanofiltration device into the hydroformylation reactor.

**13.** Process according any of the preceding claims, **characterized in that**, on exceedance of a fixed maximum concentration of the 3-methylbutanoic acid in the resulting retentate of the nanofiltration device, the recycling of the resulting retentate into the hydroformylation reactor is stopped.

**14.** Process according to any of the preceding claims, **characterized in that**, on exceedance of a fixed maximum concentration of 3-methylbutanoic acid in the feed of the nanofiltration device, one or more process parameters in the step of hydroformylating the isobutenic hydrocarbon stream in the hydroformylation reactor are modified such that the concentration of 3-methylbutanoic acid in the feed of the nanofiltration device is lowered below the fixed maximum.

**Revendications**

**1.** Procédé pour la préparation d'un mélange (2) de produits par hydroformylation technique d'un flux (1) hydrocarboné contenant de l'isobutène et pour la séparation du mélange (2) de produits obtenu, présentant les étapes suivantes :

a) hydroformyler le flux (1) hydrocarboné contenant de l'isobutène dans un réacteur (R) d'hydroformylation en présence d'un catalyseur de type complexe de métal de transition de manière à obtenir un mélange (2) de produits qui comprend au moins du 3-méthylbutanal, des produits secondaires sous forme de fraction lourde et d'acide 3-méthylbutanoïque ainsi que le catalyseur de type complexe de métal de transition ainsi que ses ligands libres,
b) séparer le mélange (2) de produits au moyen d'un dispositif (M) de nanofiltration comprenant un ou plusieurs étages de séparation sur membrane, de manière telle que le catalyseur de type complexe de métal de transition et ses ligands libres sont enrichis par rapport au 3-méthylbutanal et à l'acide 3-méthylbutanoïque dans le retentat (3) résultant du dispositif (M) de nanofiltration et de manière telle que le 3-méthylbutanal et l'acide 3-méthylbutanoïque sont à chaque fois enrichis par rapport au catalyseur de type complexe de métal de transition dans le perméat (4) résultant du dispositif (M) de nanofiltration, la concentration en acide 3-méthylbutanoïque dans le retentat (3) résultant étant inférieure à celle dans le perméat (4),
c) séparer le perméat (4) résultant du dispositif (M) de nanofiltration au moyen d'un dispositif (D) de séparation thermique comprenant un ou plusieurs étages de séparation en au moins une première fraction (5) et une deuxième fraction (6), la première fraction (5) présentant une concentration en 3-méthylbutanal plus élevée que la deuxième fraction (6) et une concentration plus basse en produits secondaires sous forme de fraction lourde et d'acide 3-méthylbutanoïque que la deuxième fraction (6),
d) recycler au moins un flux partiel du retentat (3) résultant du dispositif (M) de nanofiltration dans le réacteur (R) d'hydroformylation ;

et comprenant en outre l'étape :

- surveiller la concentration en acide 3-méthylbutanoïque dans le retentat (3) résultant du dispositif de nanofiltration et de préférence également dans le mélange (2) de produits et/ou dans le perméat (4) résultant du dispositif de nanofiltration.

**2.** Procédé selon la revendication 1, **caractérisé en ce que** lors de l'étape d'hydroformylation du flux hydrocarboné contenant de l'isobutène, un ou plusieurs paramètres de procédé sont réglés de manière telle que la concentration totale en produits secondaires sous forme de fraction lourde et d'acide 3-méthylbutanoïque est, en poids par rapport au mélange total de produits, de 30% en poids ou moins,
le ou les paramètres à régler étant de préférence choisis dans le groupe constitué par la pression, la température, le temps de séjour moyen, la composition du gaz de synthèse, la concentration en métal de transition et le rapport métal de transition-ligand du catalyseur de type complexe de métal de transition.

**3.** Procédé selon la revendication 1 ou 2, **caractérisé en ce que** l'étape d'hydroformylation du flux hydrocarboné

contenant de l'isobutène est réalisée

- à une pression dans la plage de 0,2 à 8 MPa

et/ou

- à une température dans la plage de 70 à 130°C

et/ou

- à un temps de séjour moyen dans la plage de 1 à 4 h

et/ou

- à une composition $CO:H_2$ du gaz de synthèse de 1:3 à 3:1

et/ou

- à une concentration en métal de transition dans la plage de 10 à 100 ppm

et/ou

- à un le rapport métal de transition-ligand dans la plage de 1:4 à 1:50.

**4.** Procédé selon l'une quelconque des revendications précédentes, **caractérisé en ce que**

- le métal de transition du catalyseur de type métal de transition est le rhodium

et/ou

- le ou les ligands du catalyseur de type complexe de métal de transition sont choisis dans le groupe des ligands organophosphorés.

**5.** Procédé selon l'une quelconque des revendications précédentes, **caractérisé en ce qu'**on utilise un catalyseur de type complexe de métal de transition choisi dans le groupe constitué par le rhodium et le cobalt.

**6.** Procédé selon l'une quelconque des revendications précédentes, **caractérisé en ce qu'**on utilise, dans le dispositif de nanofiltration, une ou plusieurs membranes de nanofiltration, les ou au moins une des membranes de nanofiltration présentant une rétention pour l'acide 3-méthylbutanoïque de -1 ou moins, de manière particulièrement préférée de -5 ou moins et en particulier de -10 ou moins.

**7.** Procédé selon l'une quelconque des revendications précédentes, **caractérisé en ce qu'**on utilise, dans le dispositif de nanofiltration, une ou plusieurs membranes de nanofiltration comprenant ou constituées par un ou plusieurs polymères contenant des groupes imide, le ou les polymères contenant des groupes imide étant choisis de manière telle que la rétention de la membrane de nanofiltration pour l'acide 3-méthylbutanoïque est de -1 ou moins, de manière particulièrement préférée de -5 ou moins et en particulier de -10 ou moins.

**8.** Procédé selon l'une quelconque des revendications précédentes, **caractérisé en ce qu'**on utilise, dans le dispositif de nanofiltration, une ou plusieurs membranes de nanofiltration, les ou au moins une des membranes de nanofiltration présentant une limite de séparation dans la plage de 150 à 2000 g/mole, de préférence de 200 à 600 g/mole et de manière tout particulièrement préférée de 350 à 500 g/mole.

**9.** Procédé selon l'une quelconque des revendications précédentes, **caractérisé en ce qu'**on utilise, dans le dispositif de nanofiltration, une ou plusieurs membranes de nanofiltration comprenant ou constituées par un ou plusieurs polymères contenant des groupes imide, le ou les polymères contenant des groupes imide étant choisis de manière telle que la limite de séparation de la membrane de nanofiltration se situe dans la plage de 150 à 2000 g/mole.

**10.** Procédé selon l'une quelconque des revendications précédentes, **caractérisé en ce que** l'étape de séparation du

mélange de produits obtenu par l'hydroformylation du flux hydrocarboné contenant de l'isobutène dans le dispositif de nanofiltration est réalisée

- à une température dans la plage de 10 à 150°C

et/ou

- à une pression transmembranaire dans la plage de 0,5 à 6 MPa

et/ou

- à un nombre de Reynolds entre 55 et 13.500, de préférence entre 100 et 3500 et de manière tout particulièrement préférée entre 170 et 900

et/ou

- en présence de monoxyde de carbone et/ou d'hydrogène, de préférence à une pression partielle de monoxyde de carbone d'au moins 200 kPa dans l'alimentation, dans le retentat et dans le perméat de chaque étage de séparation sur membrane.

**11.** Procédé selon l'une quelconque des revendications précédentes, **caractérisé en ce que** l'étape de séparation thermique comprend une distillation lors de laquelle la première fraction est obtenue en tant que produit de tête et la deuxième fraction en tant que produit de fond.

**12.** Procédé selon l'une quelconque des revendications précédentes, comprenant les étapes :

- hydroformyler un flux hydrocarboné contenant de l'isobutène, en présence d'un catalyseur de type complexe de rhodium présentant un ou plusieurs ligands organophosphorés, à une pression dans la plage de 0,2 à 8 MPa et à une température dans la plage de 70 à 130°C, à un temps de séjour moyen dans la plage de 1 à 4 h, à une composition $CO:H_2$ de gaz de synthèse de 1:3 à 3:1, à une concentration en rhodium dans le réacteur dans la plage de 10 à 100 ppm et à un rapport rhodium-ligand dans la plage de 1:4 à 1:50 ;
- séparer le mélange de produits résultant de l'hydroformylation au moyen d'un dispositif de nanofiltration à une température dans la plage de 10 à 150°C, à une pression transmembranaire dans la plage de 0,5 à 6 MPa, à un nombre de Reynolds entre 170 et 900 et à une pression partielle de monoxyde de carbone supérieure à 0,2 MPa dans l'alimentation, dans le retentat et dans le perméat de chaque étage de séparation sur membrane ;
- séparer par voie thermique le perméat résultant du dispositif de nanofiltration par distillation en une première fraction et une deuxième fraction, la première fraction présentant une concentration en 3-méthylbutanal plus élevée que la deuxième fraction et une concentration plus basse en fraction lourde que la deuxième fraction,
- recycler au moins un flux partiel du retentat résultant du dispositif de nanofiltration dans le réacteur d'hydroformylation.

**13.** Procédé selon l'une quelconque des revendications précédentes, **caractérisé en ce que** lors du dépassement d'une valeur maximale fixée de la concentration en acide 3-méthylbutanoïque dans le retentat résultant du dispositif de nanofiltration, le recyclage du retentat résultant dans le réacteur d'hydroformylation est interrompu.

**14.** Procédé selon l'une quelconque des revendications précédentes, **caractérisé en ce que** lors du dépassement d'une valeur maximale fixée de la concentration en acide 3-méthylbutanoïque dans l'alimentation du dispositif de nanofiltration, un ou plusieurs paramètres de procédé de l'étape d'hydroformylation du flux hydrocarboné contenant de l'isobutène dans le réacteur d'hydroformylation sont modifiés de manière telle que la concentration en acide 3-méthylbutanoïque dans l'alimentation du dispositif de nanofiltration est abaissée sous la valeur maximale fixée.

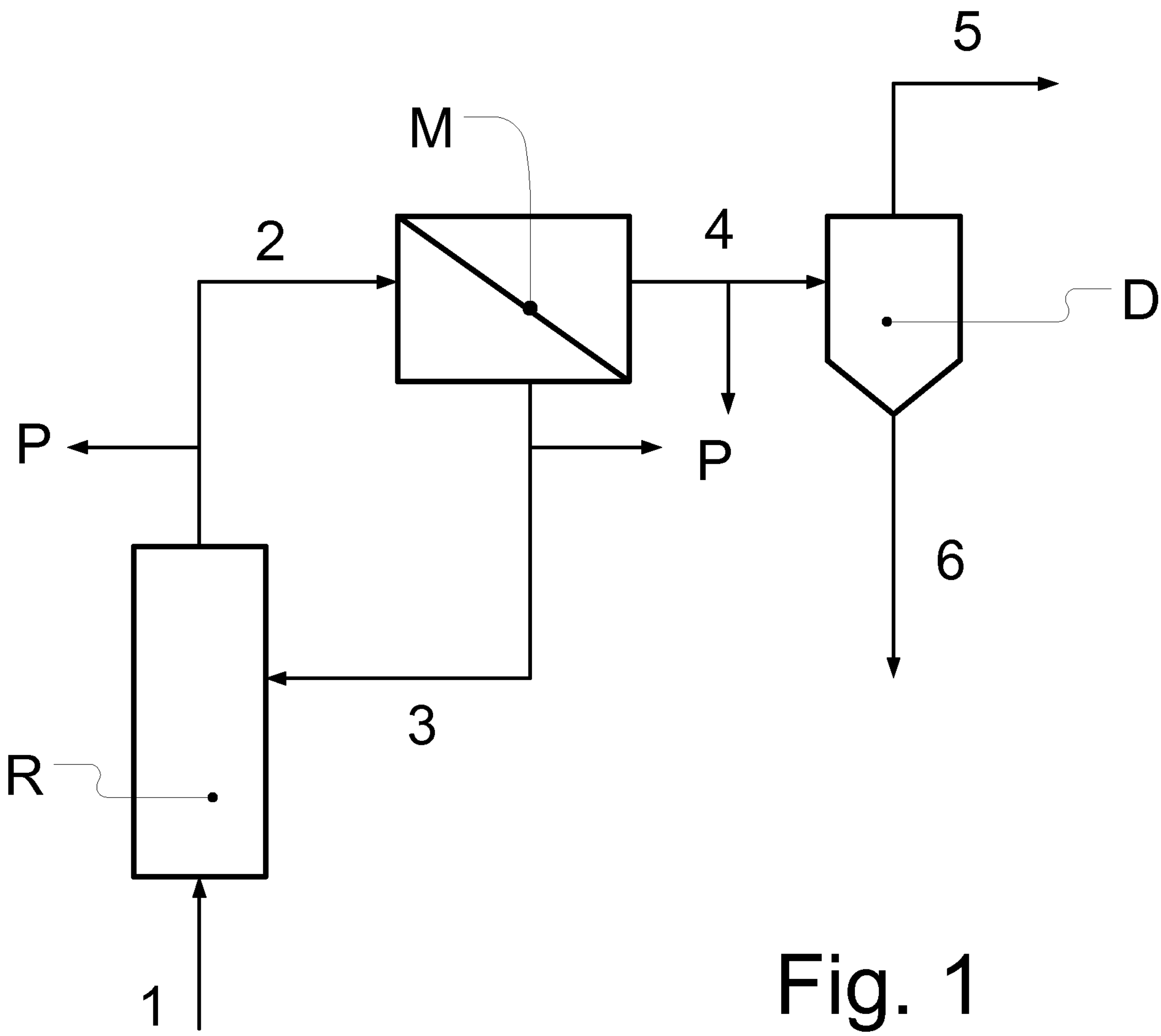


Fig. 1

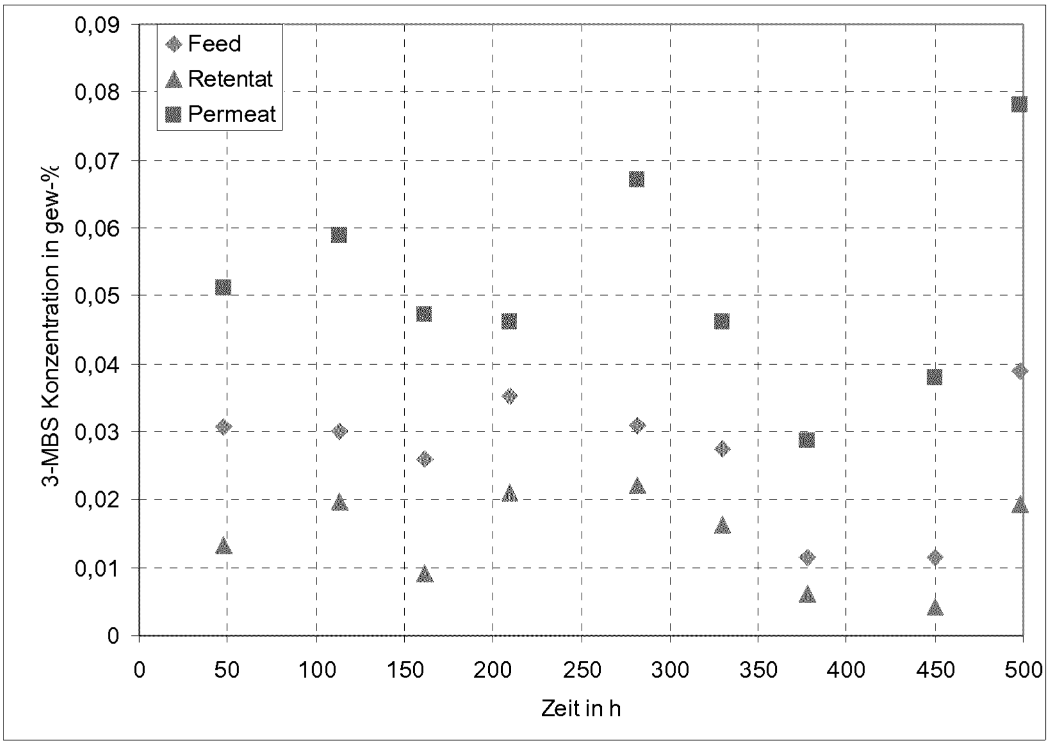
Feed
Retentat
Permeat
3-MBS Konzentration in gew-%
Zeit in h
0,09
0,08
0,07
0,06
0,05
0,04
0,03
0,02
0,01
0
0
50
100
150
200
250
300
350
400
450
500

Fig. 2

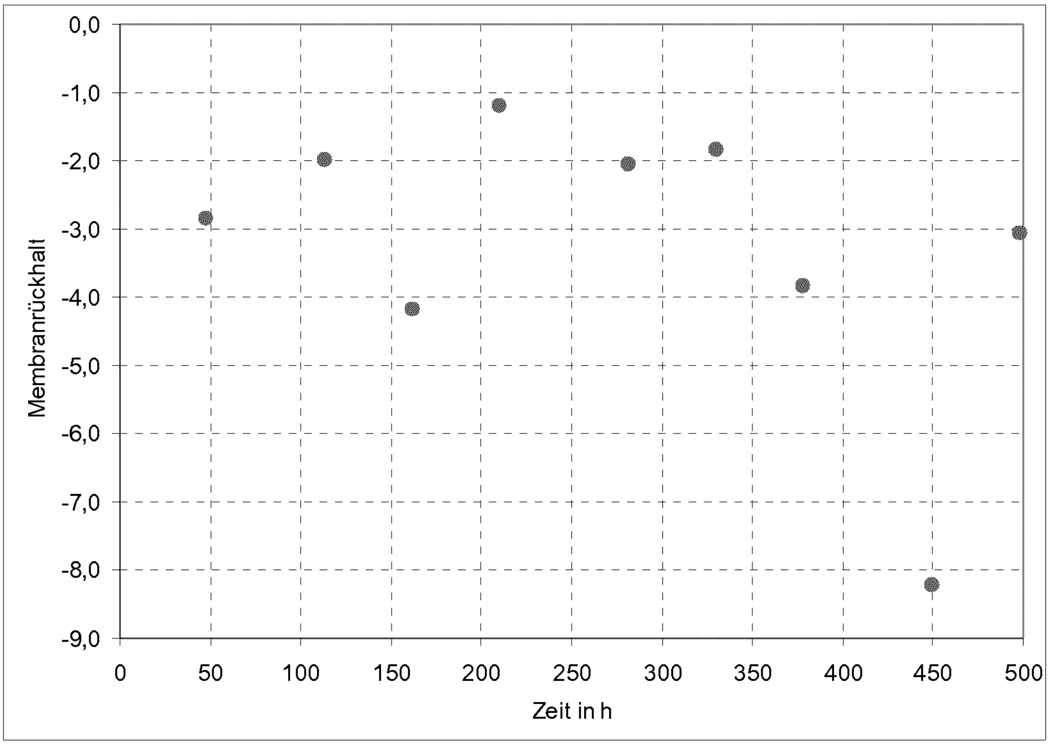
0,0
-1,0
-2,0
-3,0
-4,0
-5,0
-6,0
-7,0
-8,0
-9,0
Membranrückhalt
0
50
100
150
200
250
300
350
400
450
500
Zeit in h

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1931472 B1
- WO 2008006633 A
- WO 2010097376 A1
- WO 2010097428 A
- DE 10308111
- EP 0781166 A


### In der Beschreibung aufgeführte Nicht-Patentliteratur


- **FALBE ; JÜRGEN.** New Syntheses with Carbon Monoxide. Springer Verlag, 1980
- **PRUETT, ROY L.** *Hydroformylation. Advances in Organometallic Chemistry,* 1979, vol. 17, 1-60
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- **TOH, X.X. LOH ; A. BISMARCK ; A.G. LIVINGSTON.** In search of a standard method for the characterisation of organic solvent nanofiltration membranes. *J. Membr. Sci,* 2007, vol. 291, 120-125
- Membranes. **VON I. CABASSO.** Encyclopedia of Polymer Sience and Technlogy. John Wiley and Sons, 1987